(19) Europäisches Patentamt
European Patent Office
Office européen des brevets

(11) **EP 2 465 428 B1**

(12) **EUROPEAN PATENT SPECIFICATION**

(45) Date of publication and mention
of the grant of the patent:
**12.04.2017 Bulletin 2017/15**

(51) Int Cl.:
***A61B 5/05*** *(2006.01)*   ***A61B 6/00*** *(2006.01)*
***A61B 5/103*** *(2006.01)*

(21) Application number: **11150726.5**

(22) Date of filing: **12.01.2011**

(54) **Electromagnetic detection system and method for locating tumors/calcifications in tissues**

Elektromagnetisches Erkennungssystem und Verfahren zur Lokalisation von Tumoren/Verkalkungen in Geweben

Système de détection électromagnétique et procédé pour localiser des tumeurs/calcifications dans des tissus

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2010 EP 10195033**

(43) Date of publication of application:
**20.06.2012 Bulletin 2012/25**

(73) Proprietor: **Medielma S.r.l.**
**20145 Milano (IT)**

(72) Inventors:
• **Spagnolini, Umberto**
**20035 Lissone (MB) (IT)**
• **Bellorofonte, Carlo**
**20157 Milan (IT)**

• **Canonico, Michelangelo**
**20149 Milan (IT)**

(74) Representative: **Postiglione, Ferruccio et al**
**Gregorj S.r.l.**
**Intellectual Property**
**Via L. Muratori, 13 b**
**20135 Milano (IT)**

(56) References cited:
**EP-A1- 2 110 076**   **EP-A2- 1 264 576**
**WO-A1-01/07909**   **US-A- 6 061 589**
**US-A1- 2010 069 744**

• **"Trimprobe user manual", , 14 July 2008 (2008-07-14), XP055176452, Retrieved from the Internet: URL:www.galileoavionica.it [retrieved on 2015-03-13]**

## Description

### Technical field

This invention relates to electromagnetic detection systems for the detection of anomalies (e.g. tumors and calcifications) in living tissue, and in particular to early detection of breast, colorectal and prostatic cancer.

### Background of the invention

Over the last decades, early diagnostics has contributed to decrease the incidence rate of deaths caused by tumors. However, cancer still remains major public health problem worldwide as approximately 25% of deaths are due to cancer, and over 50% for each sex are due to prostate/breast, lung, and colon rectum (see document [1]). Advances in early diagnostic and therapy are the two leading complementary areas to reduce the incidence of deaths.

Diagnostic tools have different efficacies depending on type and location of tumors, and clinicians optimize their synergic use to minimize false-diagnosis by compounding their effectiveness. In diagnostics false-negatives induce to delay treatments that are mostly effective at early stage, while false-positive cause unnecessary discomfort to patient with redundant costs for the health system. Therefore, any research in new diagnostic devices has to improve the overall diagnostic procedure evaluated with the metric of statistics of clinical data. Early diagnostic methods for massive screening need to comply with some basic requirements for each analysis such as reliability, equipment complexity and costs, simplicity of use, and time for each diagnostic test.

Tumor is an abnormal mass of tissues surrounded by normal body tissue that has no useful function and grows at the expense of healthy tissue. Spread of malignant cells induces lethal distant metastasis with a probability that depends on the size of primary tumor (see document [6]). Generally tumors have higher water content than normal surrounding cells because of cellular necrosis, increased and irregular vascolarization, and some alteration of nutrients (see document [5]).

Since many tumors have a significant different electrical conductivity and permittivity from the normal surrounding tissues, this property is used in some diagnostic methods. Since the specific electrical properties show large difference for different tumors as namely depending on the size and the development stage of the tumor, the electromagnetic (EM) diagnostic tools basically provides the clinicians with the images of the locally altered anatomy.

Knowledge of electrical property of tissues is the basis to develop EM diagnostic methods for tumors. Topic has been of interest for over a century for several reasons, models have been developed to account for behaviours of biological tissues either normal or pathological (see document [2]). Microscopic description of the electrical properties of cells is complicated by the variety and the distribution of cell shapes, therefore a macroscopic approach is most often adopted to establish the specific conductivity and relative permittivity of biological systems. Even at macroscopic level these properties are complicated by several factors such as tissue orientation, frequency, inhomogeneity, and physiological factors.

Experimental measurements in different settings and various tissues are reliably parameterized by using the Debye model that accounts for frequency variation with relaxation times. The same model can extrapolate the macroscopic properties at high frequency (see documents [3] [4]).

The common non-invasive diagnostics are based on inner body imaging using different technologies (e.g., ultrasound, X-ray, CAT, MRI, PET) as the clinician can evaluate size and localization of the tumor from the altered anatomy.

Imaging methods based on non-ionizing EM waves have been investigated over the last decades by several microwave engineers. Since normal breast tissues are translucent to microwaves (as mostly low-water content fat tissue), the high dielectric contrast of malignant tissues compared to the surrounding area (at least 5:1) and the fairly simple geometry of the local body makes breast tumor an excellent pathology for optimizing microwave modeling and imaging (see document [7]). Active microwave (>1GHz) imaging techniques for breast tumor are based on two main methods: tomography and radar.

Document US-A-6061589 describes a microwave antenna for cancer detection system comprising co-sited resistance-loaded cross-polarized dipoles that are placed directly upon the breast. One of the dipoles is used to illuminate the breast with a very short duration pulses with large bandwidth, usually exceeding 3GHz; the other dipole is used to receive the backscattered returns. A software processes the backscattered waveforms for the purpose to image the tissues.

Document EP-A-1264576 describes a medical broad-band electromagnetic holographic imaging method. Document US-A-2009-0129652 discloses a high resolution radio frequency medical imaging and therapy system.

The goal of tomography is to recovery the dielectric property of breast from multiple views using narrowband signals (see document [7]). Ultrawideband (UWB) radar seeks to identify the presence and the location of a significant scatterer in the breast (see document [8]), and similarly to tomography multiple views can be used to image these scatterers (see document [9]).

Numerical modelling for heterogeneous (up to 20% heterogeneity) and realistic propagating medium proved that shallow (3-4cm depth) and small (below 0.5cm) tumors can be detected by UWB radar system over 4-8GHz bandwidth [8]. Since all these EM methods aim to provide an image of inner body for diagnostic, the main drawbacks are the cost for equipment/acquisition that is comparable with diagnostic imaging tools routinely employed.

There is a peak of absorption around 300-500MHz namely due to the increase of water and sodium in malignant tissues (see documents [2] and [4]). In hyperthermia this property is used to locally raise the temperature in order to induce cytotoxic effects and make malignant cells more vulnerable to ionizing radiation and chemical toxins as adjuvant to radiotheraphy and/or chemotherapy (see document [11]).

The diagnostic use of EM in the frequency range around 434MHz is for thermoacoustic tomography (see document [10]). Once again, the dielectric properties of tissues in this frequency range make the EM radiation to be absorbed differently of healthy/tumor tissues. Once radiated, the tissues expansion produces pressures waves that are measured at surface and are used to image the biologic tissues on the basis of its absorption of EM radiation.

Even if narrowband signals below 500MHz are not suited for imaging due to the lack of resolution, malignant tissues have large electrical contrasts compared to the surrounding tissues that can closely couple with radiating antenna if tumors are shallow enough (say below 5cm if permittivity ranges within 5-10 as for fat tissues), even for early-stage tumors [8].

A diagnostic use of near-field coupling is described by document WO-A-2001-07909 where is disclosed a peculiar pulse-oscillator that can shift its frequency depending on the nature of the tissues it is coupled with. However, the frequency-shift and attenuation for the set of harmonics largely depends on the degree of coupling (e.g., orientation of the device with respect to the body) and thus its use in diagnostics is not clinicians' independent.

**List of further prior art documents**

[1] A.Jemal, R.Siegel, J.Xu, and E.Ward, Cancer Statistics - 2010, Cancer J Clin. Vol. 60, pp.277-300, Sept.2010.

[2] K.R.Foster and H.P.Schwan, Dielectric properties of tissues. In: C.Polk and E.Postow eds., Handbook of Biological Effects of Electromagnetic Field. New York: CRC Press, 1996

[3] S. Gabriel, R. W. Lau, and C. Gabriel, The dielectric properties of biological tissues: III. Parametric models for the dielectric spectrum of tissues", Phys. Med., Biol., vol. 41, no. 11, pp. 2271 - 2293, 1996.

[4] K.R.Foster and H.P. Schwan, Dielectric properties of tissues and biological materials: a critical review, Crit. Rev. Biomed. Eng., vol. 17,pp. 25 - 104, 1989.

[5] P.Vaupel, F. Kallinowski, and P.Okunieff, Blood flow, oxygen and nutrient supply, and metabolic microenvironment of human tumors: a review. Cancer Res., vol.49; pp. 6449-6465, Dec.1989.

[6] J.S.Michaelson et al., Predicting the survival of patients with breast carcinoma using tumor size, Cancer vol. 95, n.4, pp. 713-723, Aug.2002.

[7] E.C.Fear, S.H.Hagness, P. M. Meaney, M. Okoniewski, and M. A.Stuchly, Enhancing breast tumor detection with near field imaging, IEEE Microwave Mag., vol. 3, pp. 8-56, Mar 2002

[8] S. C. Hagness, A. Taflove, and J. E. Bridges, "Two-dimensional FDTD analysis of a pulsed microwave confocal system for breast cancer detection: fixed-focus and antenna-array sensors", IEEE Trans. Biomed. Eng., vol. 45 , pp. 1470 - 1479, 1998.

[9] E. C. Fear, X. Li, S. C. Hagness, and M. A. Stuchly, "Confocal microwave imaging for breast cancer detection: Localization of tumors in three dimensions", IEEE Trans. Biomed. Eng., vol. 49, no. 8, pp. 812 - 822 , 2002.

[10] R.A.Kruger, K.K.Kopecky, A.M.Aisen, D.R.Reinecke, G.A.Kruger, W.L.Kiser Jr, Thermoacoustic CT with radio-waves: a medical imaging paradigm, Radiology vol.211, n.1, pp.275-278, April 1999

[11] P. Wust , B. Hildebrandt, G. Sreenivasa, B. Rau , J. Gellermann , H. Riess, R. Felix and P. M. Schlag "Hyperthermia in combined treatment of cancer", Lancet Oncol., vol. 3, pp. 487 2002.

[12] C.Bellorofonte, C.Vedruccio, P.Tombolini, M.Ruoppolo, A.Tubaro, Non-invasive detection of prostate cancer by electromagnetic interaction, European Urology, vol.47, pp.29-37, 2005.

[13] A.Vannelli, E.Leo, L.Battaglia, E.Poiasina, Diagnosis of rectal cancer by electromagnetic interactions: preliminary results. Desease of the Colon & Rectum, vol.52, n.1, pp.162-166, 2009.

[14] Q.Fang, P.M.Meaney, K.D.Paulsen, The multidimensional phase unwrapping integral and applications to microwave tomographical image reconstruction. IEEE Trans. On Image Processing, vol. 15, n.11, pp.3311-3324, Nov..2006.

[15] H. Hinrikus, J.Riipulk, Microwave imaging. Wiley Encyclopedia of Biomedical Engineering, J.Wiley and Sons Ed., Apr.2006.

**Summary of the invention**

It is an object of the invention to provide a system for anomaly tissues detection that reliably allows to obtain an early-detection and localization of anomalies and does not require a specialized expertise in interpretation of body-images.

The object of the invention is obtained by the detection system of claim 1. Particular embodiments of the detection system are defined by the dependent claims 2-14. A further object of the invention is a detection method as defined by claim 16.

**Brief description of the drawings**

Further characteristics and advantages will be more apparent from the following description of preferred embodiment and of its alternatives given as a way of an example with reference to the enclosed drawings in which:

Figure 1 schematically shows a first embodiment of a tissue anomaly detection system including a probe antenna device and a receiving device;

Figure 2 shows an example of said probe antenna device;

Figure 3 schematically shows said detection system operating on a patient;

Figure 4 depicts a simplified scattering phenomena employed by said detection system;

Figure 5A shows schematically an example of probe antenna device with multiple frequencies;

Figure 5B shows schematically a demodulator for dual polarized antenna system with a quadricorrelator, employable by said receiving device;

Figure 6 shows a particular example of said detection system employing multiple dual-polarization antennas;

Figure 7A illustrates schematically propagation in a medium included between said antenna probing device and said receiving device;

Figures 7B shows an example of the time varying medium response on one link;

Figure 8A depicts an example of a spectral view of a signal generated by one antenna probing device;

Figure 8B depicts an example of a spectral view of a signal received by the receiving device;

Figure 9A illustrates an example of a medium response for a first operating method;

Figure 9B shows an example of a medium response for a second operating method;

Figure 10A shows a behavior of scattering power of tumor tissues;

Figure 10B shows a behavior of scattering power of calcification tissues;

Figure 11A shows a scattering energy table for prostate analysis without tumor while Figure 11B refers to the presence of tumor;

Figure 11C shows an elevation view of power scattering patterns for prostate analysis without tumor while Figure 11D refers to the presence of tumor;

Figure 11E shows schematically the a plurality of scattering energy tables to be displayed by a display of the detection system;

Figure 12 shows an example of a software architecture employable by the detection system.

**Detailed description**

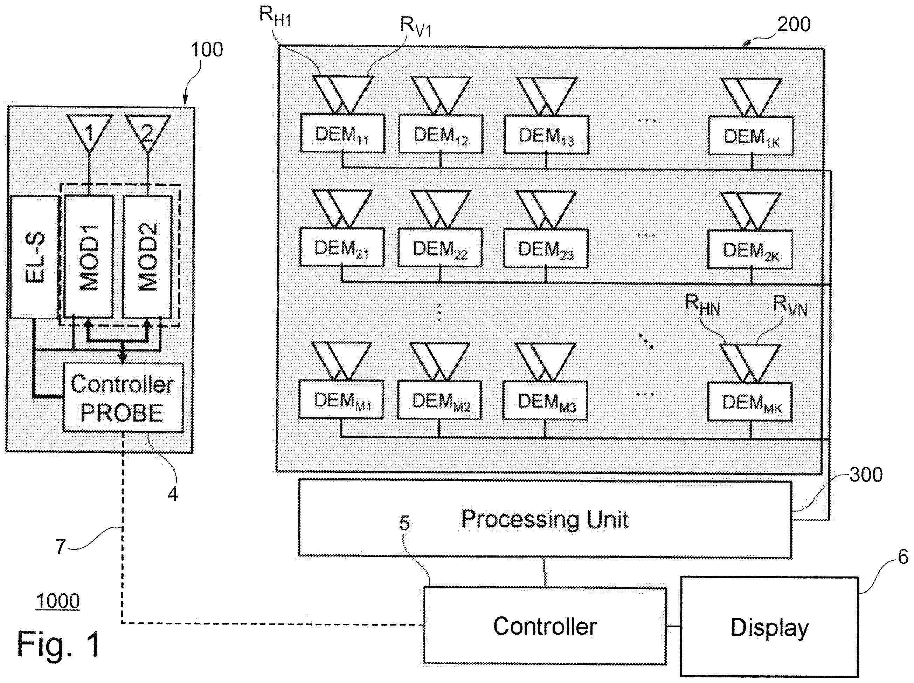

In figure 1 a first embodiment of a tissue anomaly detection system 1000 (hereinafter also called "detection system") is schematically shown. The detection system 1000 is structured to detect anomalies in tissues of human or animal bodies. The anomalies can be, as an example, tumors and/or calcifications. Particularly, tumors in early stage (e.g. malignant breast, prostatic and colorectal tumors) can be detected by the system 1000.

The detection system 1000 includes a probe antenna device 100, a receiving device 200 and a processing module 300. The probe antenna device 100 can be, particularly, hand-held and is structured to radiate an incident radio-frequency signal. Particularly, the probe antenna device 100 includes a first antenna 1 connected to a first modulating module MOD1. The probe antenna device 100 is provided with at least a second antenna 2 connected to a second modulating module MOD2. The probe antenna device 100 is also provided with a first controller module 4 (Controller PROBE) configured to control the first and second modulating modules MOD1-MOD2. The probe antenna device 100 may be provided with variable-gain amplifiers placed between each of the modulating module MOD1-MOD2 and the each of the first and second antennas 1 and 2.

The probe antenna device 100 includes a variable frequency oscillator EL-S that operates as electromagnetic wave source that is controlled by the first controller module 4 and is connected with the first and second modulators MOD1-MOD2. It is also observed that in accordance with another example the electromagnetic wave source EL-S, the first controller module 4, the first and second modulating modules MOD1 and MOD2 can be external to the probe antenna device 100.

The detection system 1000 operates with electromagnetic waves at a frequency below 700 MHz. According to an example, the electromagnetic source EL_S is configured to generate radio-frequency signals at K frequencies $f_1$, $f_2$,..., $f_K$ properly chosen to be within a predefined frequency span around a central carrier frequency $f_0$ with 350MHz $<f_0<$ 550MHz. The maximum frequency span of the K frequencies might be below 100MHz, typical values are 1MHz, 5MHz, 10MHz. Moreover, according to a second embodiment that will be described in detail later, the first and second modulating modules MOD1-MOD2 can include a respective FDM (Frequency Division Multiplexing) block acting on each of said radio-frequency signals at K frequencies $f_1$, $f_2$,..., $f_K$. Particularly, the number of frequencies can be K=201, even K=31, K=7 and degenerate to K=1 in some cases.

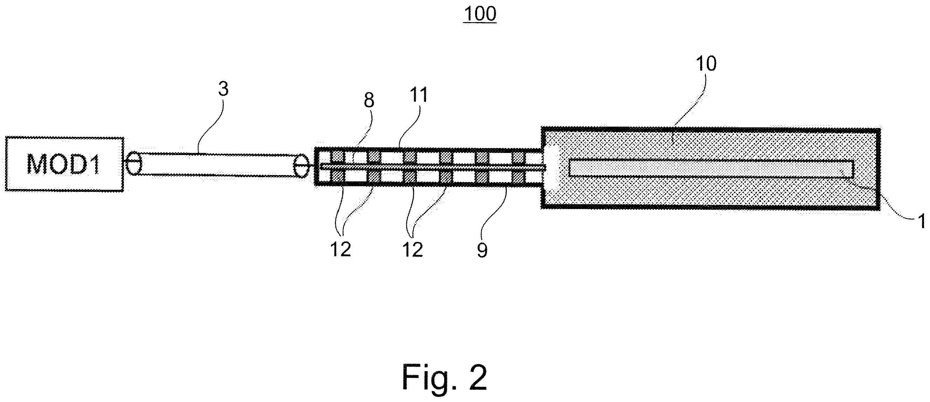

Figure 2 shows a particular embodiment of the probe antenna device 100 employing a single antenna, i.e. the first antenna 1 which can be, according to the example, a dipole antenna operating at half the wavelength. Moreover, the first antenna 1 operates to generate a linearly polarized electromagnetic field.

The probe antenna device 100 of figure 2 comprises a case 9 made, as an example, of dielectric material such as plastic, which defines an active radiating portion 10, including the first antenna 1, and a handle portion 11. The handle portion 11 includes electromagnetic waves absorbing elements 12 (e.g. ferrite rings). The absorbing elements 12 are an example of balun employable to minimize any influence onto the radiating portion 10 of any support used to support the probe antenna device 100, such as the hand of a user. In accordance with the particular example shown in figure 2, the first antenna 1 is connected to the first modulating module MOD1 by a suitable first cable 8 (as an example, passing trough the handle portion 11 and the ferrite rings 12) and possibly a second cable 3, external to the case 9. It is noticed that the probe antenna device 100 generates non-ionizing radiation.

It is also observed that the first antenna 1 is used in a fixed radiation frequency range. To the purposes of the present invention with the wording "fixed radiation frequency range" it is meant that the radiation frequency range of the antenna is substantially independent either from the interaction with the radiated tissue and the position variations of the first antenna 1. As an example, the first antenna 1 operates in the following radio-frequency range: 320MHz-390MHz. It is observed that the considerations made for the first antenna 1 can made also for the second antenna 2.

The probe antenna device 100 includes two antennas, the first antenna 1 radiates electromagnetic waves having a first linear polarization and the second antenna 2 radiates electromagnetic waves having a second polarization different from the first linear polarization. As an example, the first linear polarization is a horizontal polarization H and the second linear polarization is a vertical polarization V. The pair of transmitting antennas in probe 100 forms a dual polarization-antenna unit. Moreover, according to a particular embodiment, the detection system 1000 can comprise a second controller module 5 connected, via a wired or wireless link 7 to the first controller module 4, and also connected to the processing unit 300 and a display 6.

Referring back to figure 1, the receiving device 200 comprises at least a receiving antenna unit including two receiving antennas $R_{H1}$-$R_{V1}$. Preferably, the receiving device 200 includes a plurality of receiving antenna units each including two antennas and forming a receiving array $R_{H1}$-$R_{VN}$. Each antenna of a pair of receiving antennas $R_{Hi}$ - $R_{Vi}$ are configured and oriented to receive electromagnetic waves according to orthogonal linear polarizations.

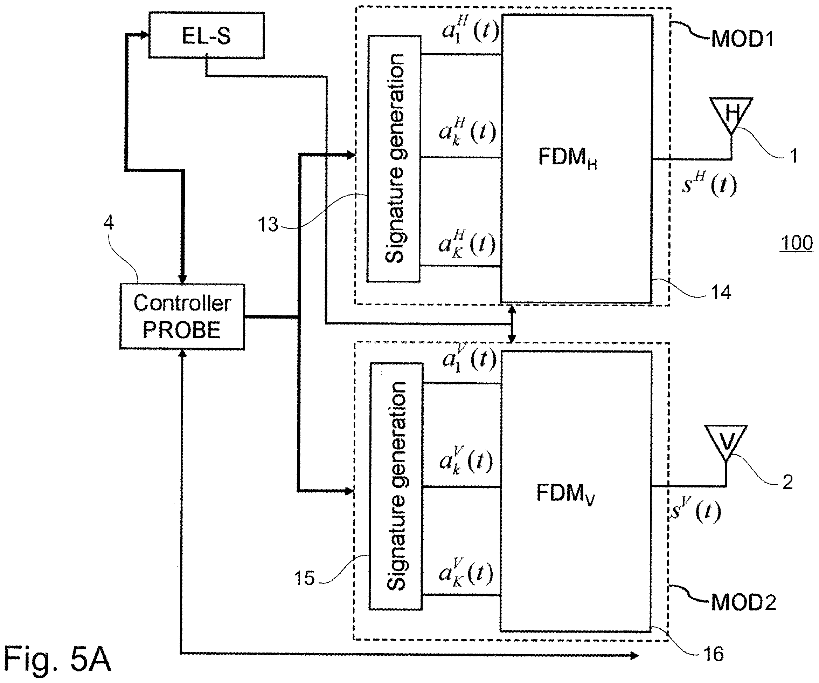

The receiving device 200 includes at least a demodulator module $DEM_{11}$ and, in accordance with the embodiment described, includes a plurality of demodulator modules $DEM_{11}$ - $DEM_{MK}$ each associated with a respective pair of receiving antennas $R_{Hi}$ - $R_{Vi}$. Particularly, as it will be described with reference to Figure 5A the second embodiment, if FDM modulating modules MOD1 and MOD2 are employed, each demodulator $DEM_{11}$ - $DEM_{MN}$ can include at least an IFDM (Inverse FDM) demodulator.

The receiving device 200 and the processing module 300 are structured to analyse from the resulting data an electrical field power scattering versus frequency pattern and detect said anomaly associated with nulls or minima in said pattern.

## First embodiment of an operation method

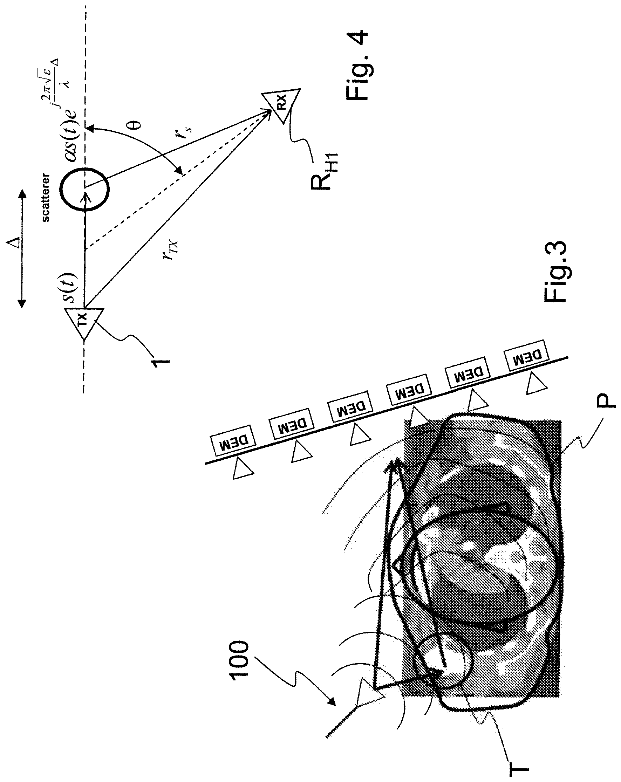

A first embodiment of operation of the detection system 1000 will be described herein after also with reference to figure 3 showing a patient P, having a tumor region T, and schematizations of the probe antenna device 100 and the receiving device 200.

The probe antenna device 100, which acts as diagnostic probe, is moved (e.g. hand-held by a clinician) and is positioned in intimate contact with the patient's skin. The continuous movement of the probe antenna device 100 aims to let the user to locate the position on the skin where the radiation pattern becomes complex, if any, being an indicator of anomalous resonance with tumor or calcification tissues.

The probe antenna device 100 generates an electromagnetic field (i.e. an incident signal) that locally couples in near-field with the tissues in contact. As an example, with reference to the prostatic tumor it is observed that around the perineum the area is electromagnetically very heterogeneous and cannot be described by just simple macroscopic values. However, for the sake of reasoning, by assuming a relative permittivity at 434MHz ranges between 7 and 12 as for mixed fat and bones, the wavelength in the body is about 19-25cm. Prostate size is 3-4cm is embedded within 3-4cm from the perineum position where the clinician has placed the probe antenna device 100 and it is in the near-field.

More specifically, "near-field" means that the distance D between the radiating antenna (e.g. the first antenna 1) and the target in-homogeneity is shorter than the wavelength used. Particularly, the distance D can be lower than 1/5 of the wavelength, preferably, lower than 1/10 of the wavelength or, more preferably, lower than 1/20. Near-field operation enables a coupling with malignant tissues that is much higher than those normally achievable by conventional far-field systems used for microwave imaging.

The cancerous cells in depth tissues of the tumor region T are excited from radio-frequency with large wavelength, this excitation generates a secondary electromagnetic field (i.e. an induced signal). The incident electromagnetic field and the induced electromagnetic field generate a scattered electromagnetic field (scattered signal). Particularly, since the tumor T is in close distance from the probe antenna device 100 in term of wavelength, the scattered field is a complex combination of incident field and the one generated by heterogeneous tissues with tumor (if present). The closer is the probe antenna device 100 to the tumor T embedded into heterogeneous tissues, and the stronger is the coupling with the tumor, this in turn generates a more complex backscattering pattern with multiple scattering nulls.

It is noticed that the electromagnetic properties of malignant tissues are altered showing a large dielectric discontinuity and conductivity with respect to the surrounding tissue. The scattered electromagnetic field when the probe antenna devices 100 is in close interaction with these inhomogeneities shows complex interference patterns with deep nulls. It is observed that the backscattering pattern is less complex and less structured when tissues are less heterogeneous such as for no-tumors.

The scattered signal is received by the receiving antennas of the receiving device 200 and it is equivalent to a modulated signal that is suitably demodulated by the demodulation modules $DEM_{11}$-$DE_{MK}$. A digital demodulated signal is than fed to the processing unit 300. It is observed that the receiving device 200 is placed in such a way that it is in far-field from the patient, typically more than one or two wavelength in distance, say 1-3meters apart.

In particularly, each receiving antenna $R_{H1}$-$R_{VN}$ is connected to an amplifier which transfers the amplified received signals to the respective demodulator $DEM_{ii}$ that is synchronized with the first and second modulating modules MOD1-MOD2 in term of phase/frequency and modulating signal. All the demodulators $DEM_{ii}$ acquire the backscattered field synchronously, and the synchronization of each demodulator with the probe antenna device 100 can be either guaranteed by a physical connection 7 between the probe antenna device 100 and the second controller module 5, or by a wireless link, or can be self-recovered as in conventional receiver systems using known methods.

The demodulated signals from all the receiving antennas together with the corresponding polarization are transferred to the processing module 300 that applies processing algorithms.

The presence of nulls or minima (below a threshold) in the scattering field is associated with the presence of tissue anomalies. The processing unit 300 allows running algorithms to localize the scattering nulls or minima in the pattern and it provides an indication to the user (as an example, on the display 6) who is so informed that the probe antenna device 100 is in contact with a region of the patient's body wherein there is an in-homogeneity such as the tumor T.

Therefore, the operation method includes a step of moving the probe antenna device 100 until the pattern of the backscattering nulls measured reaches some degree of complexity.

Moreover, the processing unit 300 can analyse time-varying effects at nulls for diagnostic purposes in order to provide reliable indicators on the nature of the in-homogeneity.

Figure 4 refers to a simplified situation that allows illustrating the scattering phenomena employed by the detection system 100. The first antenna 1 is coupled with a scattering point T that represents the tumor at distance $\Delta << \lambda$ that re-radiates the same signal scaled according to a coupling factor $\alpha (|\alpha| \leq 1)$ and with phase delay that depends on distance.

The signal received by a receiving antenna $R_{H1}$ at distance r is just the interference arising from the direct and scattered ray. If distance $r >> \Delta$ the power vs. angle for the backscattered radiation pattern is independent of r. Namely, the angular position of the scattering nulls depends on the phase-delay of the scattered (here it depends on distance $\Delta$) while the coupling factor $\alpha$ changes the depth of null (here changes from $\alpha=1$ to $\alpha=0.8$). Interference analysis of null scattering has a magnification effect on coupling $\alpha$ as small changes in $\alpha$ result in a large variation of attenuation when evaluated at minima of backscattering pattern.

## Second embodiment of the detection system

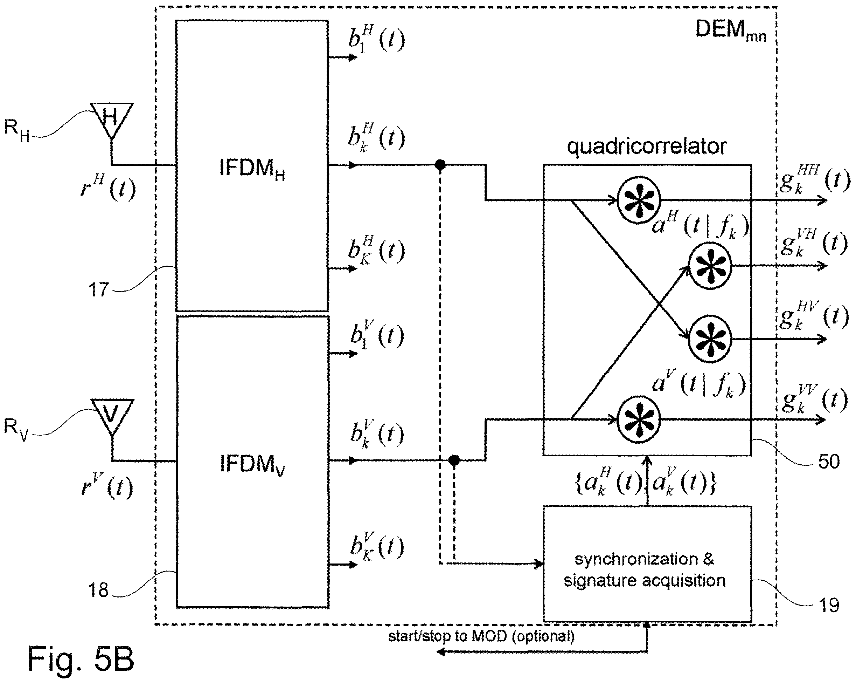

Figures 5A and 5B refer to a second embodiment of the tissue anomaly detection system 1000 and show particular embodiments of the probe antenna device 100 (Figure 5A) and of a demodulator DEMmn (Figure 5B) included in the receiving device 200. Modules or components identical or analogous to the ones above described are represented in the figures by the same reference numbers.

The probe antenna device 100 of Figure 5A has an array of dual-polarization antenna units, each provided with the first antenna 1 radiating an electromagnetic field having the horizontal polarization H and the second antenna 2 radiating an electromagnetic field at the vertical polarization V. Particularly, in Figure 5A a single dual-polarization antenna unit comprising the first and second antenna 1 and 2 is employed.

The first modulating module MOD1 includes a first signature generation block 13 connected to a first FDM modulating module 14. The second modulating module MOD2 includes a second signature generation block 15 connected to a second FDM modulating module 16. The first and second signature generation blocks 13 and 15 are structured to generate modulated signal vectors to be provided to the corresponding first and second FDM modulating modules 14 and 16.

The demodulator DEMmn (Figure 5B) is connected to a first receiving antenna $R_H$ and a second receiving antenna $R_V$ respectively connected to a first IFDM demodulation block 17 and a second IFDM demodulation block 18, included into the demodulator DEMmn. Output ports of the first and second IFDM demodulation blocks 17 and 18 are connected to a transfer matrix element computational block 50 which is, particularly, a quadricorrelator.

Moreover a synchronization and signature acquisition block 19 manages the synchronisation of the demodulator $DEM_{mn}$ and the first and second modulating modules MOD1-MOD2 in term of phase/frequency and modulating signal. The second controller module 5 5 (figure 1) provides the reference modulating signal and synchronization signals to be used by the synchronization and signature acquisition block 19.

**Examples of processing algorithms**

Reference is made, particularly, to the second embodiment of the detection system 1000, as above described with reference o figures 5A and 5B.

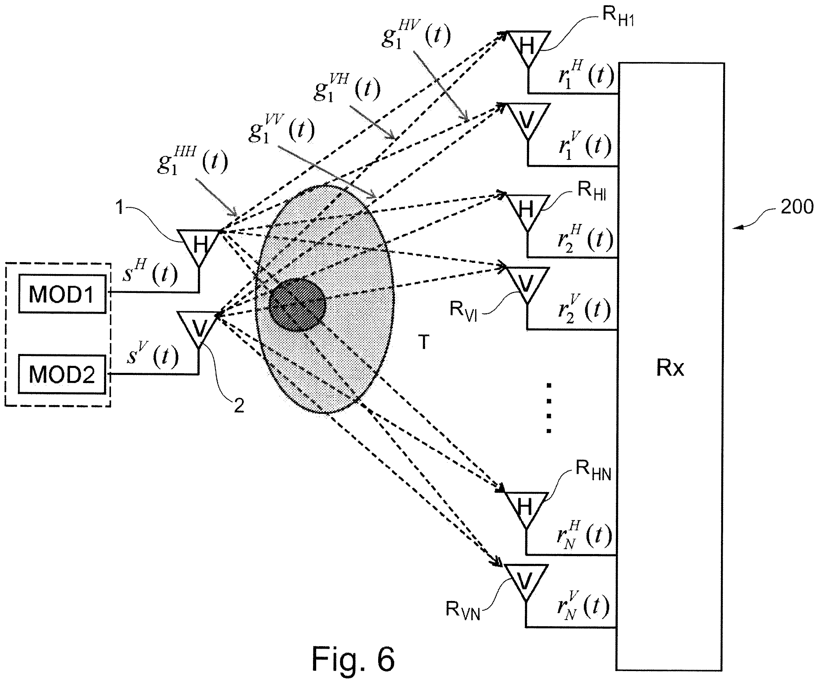

Moreover, reference will be also made to the schematization of the detection system 1000 shown in Figure 6. The receiving device 200 shown in Figure 6 includes a two dimensional array with 2N receiving antennas over rectangular grid, the inter-element spacing is typically below the wavelength (e.g. 10 cm). Each dual-polarization antenna unit ($R_{H\ell}$, $R_{V\ell}$) contains cross-polarizations and acts as a 2X2 MIMO (Multi Input Multi Output) system with 4 channel responses for the HH, NV, VH, VV links, in total there are 4N equivalent links.

Since the biological propagating medium is influenced by the radiation, the equivalent channels are time-varying according to the system settings and tissue properties.

The first and second antennas 1 and 2 transmit incident signals over H and V polarization; the incident signal are arranged in a vector $s(t)$:

$$\mathbf{s}(t) = [s^H(t), s^V(t)]^T \qquad (1)$$

The signal received by an $\ell$-th receiving link based on the antennas $R_{H\ell}$ and $R_{V\ell}$ is expressed as:

$$\mathbf{r}_\ell(t\,|\,\tau) = \begin{bmatrix} r_\ell^H(t\,|\,\tau) \\ r_\ell^V(t\,|\,\tau) \end{bmatrix} = \underbrace{\begin{bmatrix} g^{HH}(t\,|\,\tau,\ell) & g^{VH}(t\,|\,\tau,\ell) \\ g^{HV}(t\,|\,\tau,\ell) & g^{VV}(t\,|\,\tau,\ell) \end{bmatrix}}_{\mathbf{G}(t|\tau,\ell)} * \mathbf{s}(t) \qquad (2)$$

Where scattering matrix $\mathbf{G}(t|\tau,\ell)$ is the 2X2 transfer function in time-domain for the $\ell$-th link and time variations of the propagating media are accounted by the temporal factor $\tau$, symbol "*" denotes the convolution.

Let the receiving antenna index and the $\ell$-th demodulators be neglected by considering one transmitting/receiving link. Since the position of scattering nulls of time-varying transfer function matrix $\mathbf{G}(t|\tau)$ (or any of its elements) to be used as diagnostic depends on the wavelength (or equivalently on frequency), the signals of expression (1) generated by the probe antenna device 100 can be composed for instance by a superposition of a set of narrowband signals that are a mutually non-interfering set of K frequencies $f_1$, $f_2$,..., $f_K$ properly chosen to be within a predefined frequency span around the carrier frequency $f_0$ with 350MHz $<f_0<$ 550 MHz.

This multiple frequency modulation is carried out by the first FDM modulating module 14 and the second FDM modulating module 16 (Figure 5A), included into each first and second modulating modules MOD1-MOD2, that separately modulates both H and V signals by $FDM_H$ and $FDM_V$ techniques, respectively using the known concept of filter-bank modulation employed in frequency division multiplexing systems.

The bandwidth Bw of each of these narrowband components is small enough to use the assumption that channel response as result of the interaction with body can be considered as constant within the bandwidth Bw provided that 1/Bw is below the relaxation times of biological tissues (typical value Bw>1KHz).

Bandwidth Bw and frequency raster $f_1, f_2, ..., f_K$ have different combinations, for instance they can be either chosen to have non-overlapping narrowband signals ($Bw<<|f_k-f_{k-1}|$) or orthogonal signals ($Bw\sim f_k-f_{k-1}|$) as for OFDM (Orthogonal FDM) systems, the difference is in the way narrowband signals are generated (by FDM) and demodulated (by IFDM) without changing the diagnostic properties of the device at hand.

The frequency decomposition decouples the scattering measurements of a specific attenuation on a frequency by frequency basis from the slow-varying changes in electrical properties of tissues accounted by time $\tau$. This approaches resembles the short-time Fourier transform (STFT) for the analysis of non-stationary processes except that here is applied to an active system used for diagnostics.

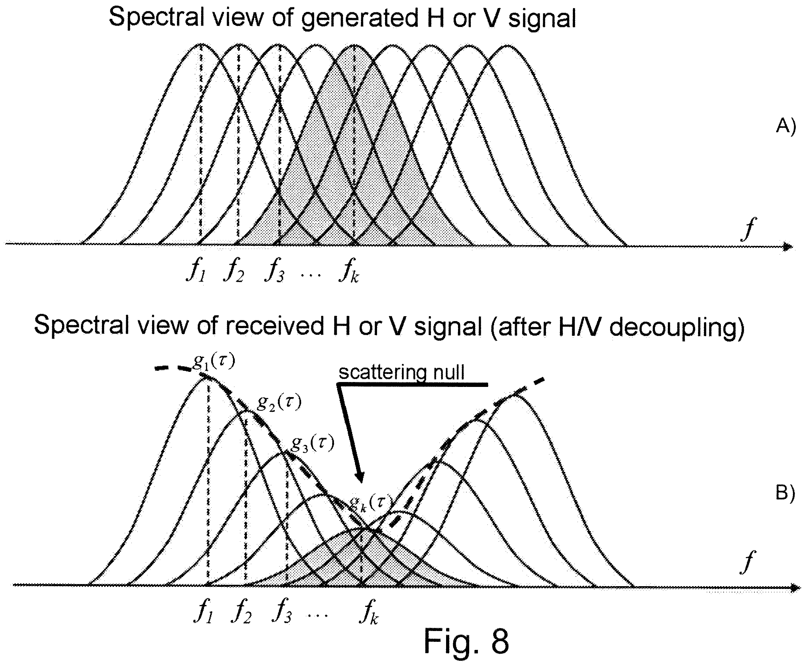

Figure 8A shows an example of a spectral view of the transmitted power vs. frequency for any of the HH, VH, HV, VV link (constant $\tau$).

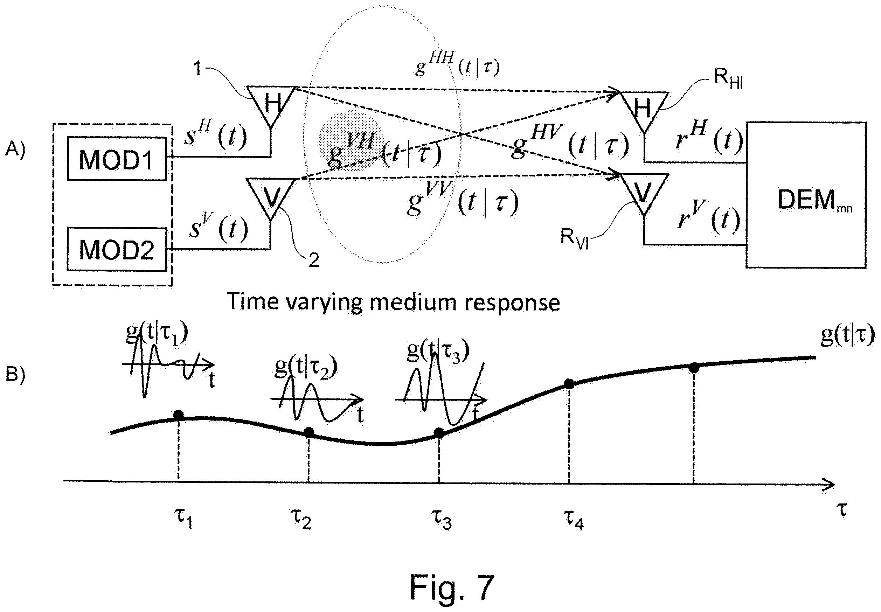

Reference is now made to the 2x2 MIMO link schematically shown in Figure 7; each of the narrowband waveform for both polarizations is modulated by the corresponding first and second modulating modules MOD1-MOD2 by the following amplitude modulating signal vector (also referred as signature):

$$\mathbf{a}_k(t) = [a_k^H(t), a_k^V(t)]^T \qquad (3)$$

different for each of the frequency $f_k$ around the carrier frequency $f_0$. The components of the modulating vector are generated by the above mentioned first and second signature generation blocks 13 and 15 (Figure 5B).

Therefore, the signals radiated by the probe antenna device 100 can be expressed by:

$$\mathbf{s}(t) = \mathrm{Re}\left\{ \sum_{k=1}^{K} \mathbf{a}_k(t) \times \exp(j2\pi(f_0 + f_k)t) \right\} \qquad (4)$$

here it is used the equivalent complex notation. The choice of the H and V signatures $a_k^H(t), a_k^V(t)$ should guarantee to estimate the kth spectral component of the four elements of the complex valued scattering matrix

$$\mathbf{G}_k(\tau) = \begin{bmatrix} g_k^{HH}(\tau) & g_k^{VH}(\tau) \\ g_k^{HV}(\tau) & g_k^{VV}(\tau) \end{bmatrix} = \int \mathbf{G}(t|\tau) \times \exp(-j2\pi f_k t)dt \qquad (5)$$

by decoupling H and V components using standard approaches such as the maximum likelihood approach. Signatures are repeated periodically with period T (typical value T=10-100ms) that is the sampling period of the time-varying scattering matrix in equation (5). The H and V signatures are mutually orthogonal within one period T, namely the following convolutive relationship should hold:

$$\mathbf{a}_k(t) * \mathbf{a}_k^T(-t) = \int \begin{bmatrix} a_k^H(\xi)a_k^H(t+\xi) & a_k^H(\xi)a_k^V(t+\xi) \\ a_k^V(\xi)a_k^H(t+\xi) & a_k^V(\xi)a_k^V(t+\xi) \end{bmatrix} d\xi = E_a \begin{bmatrix} \delta(t) & 0 \\ 0 & \delta(t) \end{bmatrix} \qquad (7)$$

where $E_a$ is the energy of the signature (to simplify, all the signatures have the same energy) and the Dirac function $\delta(t)$ denotes that the signatures have ideal autocorrelation properties. In practice, it is enough that cross-correlation $\int a_k^V(\xi)a_k^H(t+\xi)d\xi$ is below the isolation between H and V pairs in transmitting antennas 1 and 2, and the receiving antennas $R_{H\ell}$ and $R_V\ell$.

## Example of practical selection of signatures

In addition to the constraints of mutual orthogonality and ideal autocorrelation properties as in expression (7), the choice of H and V signatures $a_k^H(t)$ $a_k^V(t)$ are designed to modulate in a controlled way the amount of radiofrequency energy that are transferred to tissues once irradiated from probe 100. It is noticed that power or energy radiated over short time makes the scattering matrix $\mathbf{G}(t|\tau)$ be time-varying as result of the change in electromagnetic properties of tissues because of heating or any relevant ionic adjustment of biological tissues. The total power over T is the sum of H and V power over all the K frequencies: $P_{TX} = KE_a/T$, its value is intentionally modulated from signatures and/or period T for diagnostic purposes.

Such a very small change in electrical properties of in-homogeneity can be appreciated in the backscattering field only from the inspections of the attenuation behaviour vs. time at the scattering nulls (or minima) of the scattering matrix elements over a time-scale of 1-2 seconds where we can assume that inspecting clinician can hold the same position of the probe antenna device 100 without any meaningful change (say within 1-2cm).

According to these particular constraints related to the diagnostic method, two practical examples are proposed below referred to as "spread spectrum method" or "flip-flop method".

Spread Spectrum method

The signatures $a_k^x(t)$ (where x denotes H or V polarization) can be chosen as a set of modulating sequences based on the composition of binary codes $\{c_k^x[1], c_k^x[2], ..., c_k^x[L]\}$ with $c_k^x[\ell] = \pm 1$ selected from any known orthogonal set (e.g., Hamming, Hadamard) as for spread-spectrum and CDMA (Code Division Multiple Access) communication systems.

The signature $a_k^x(t) = \sum_{\ell=1}^{L} c_k^x[\ell] \times rect_{T_a}(t - \ell T_a)$ is a set of unit amplitude delayed square waveforms all with the same duration $T_a \ll T$ so that $LT_a \le T$ for all the frequencies. Period $T_a$ is chosen to guarantee the non-interfering property between each pair of the narrowband signals that can be chosen to be $|f_i - f_j| > 1/T_a$ for every $f_i \ne f_j$.

Flip-Flop system

Another choice is to alternate the activation over the first antenna 1, polarization H, and the second antenna 2, polarization V, (flip-flop generation) so that for any instantaneous value it is $a_k^H(t)a_k^V(t) = 0$.

A simple (but not unique) solution is to choose $a_k^H(t) = 1$ for $0 < t < T_a$ and $a_k^V(t) = 1$ for $T_a < t < 2T_a$ so that the total power is $P_{TX} = KT_a/T$ and any variation of the activation period $T_a$ rules the power transferred to the tissues.

The Flip Flop method is the preferred choice when the environmental interference is low, while spread spectrum method is more immune to interference due to the well known property of despreading (or matched filtering) used at receiver DEM for interference mitigation.

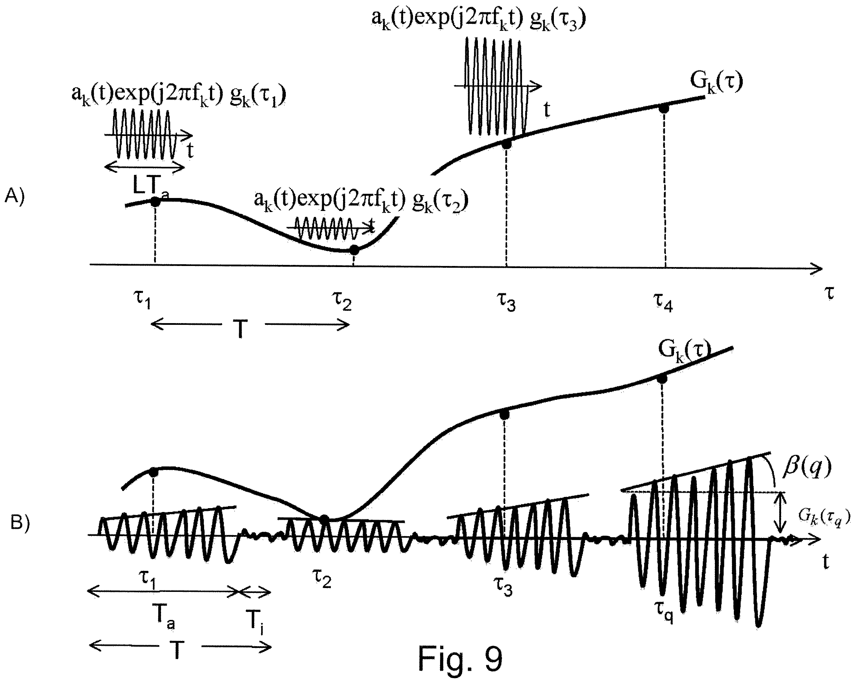

However, the signature of spread spectrum method allows obtaining an average of the time-varying performance of the transfer function over the period $LT_a$ while the Flip Flop method allows measuring the local variation. Figure 9A illustrates the response for the spread spectrum method and Figure 9B shows the response for the Flip Flop method in case of one link of the transfer matrix (either HH, HV, VH, VV).

The signal received at the receiving device 200 here for any pair of receiving antennas can be expressed as:

$$\mathbf{r}(t|\tau) = \begin{bmatrix} r^H(t|\tau) \\ r^V(t|\tau) \end{bmatrix} = Re\left\{ \sum_{k=1}^{K} \begin{bmatrix} b_k^H(t|\tau) \\ b_k^V(t|\tau) \end{bmatrix} \times \exp(j2\pi(f_0 + f_k)t) \right\} + \begin{bmatrix} n^H(t) \\ n^V(t) \end{bmatrix} \quad (8)$$

with $\mathbf{b}_k(t|\tau) = [b_k^H(t|\tau), b_k^V(t|\tau)]^T$, while $[n^H(t), n^V(t)]^T$ accounts for any system noise or environmental impairments. The base-band equivalent model for complex valued signals

$$\mathbf{b}_k(t|\tau) = \mathbf{G}_k(\tau) * \mathbf{a}_k(t) \quad (9)$$

The base-band signal is obtained from an estimate $\hat{\mathbf{b}}_k(t|\tau)$ (i.e., in general $\hat{\mathbf{b}}_k(t|\tau) \ne \mathbf{b}_k(t|\tau)$) after the received signals are decoupled onto the spectral components using the filter-bank demodulation of IFDM block for H and V components.

The estimate of the complex-valued transfer matrix $\hat{\mathbf{G}}_k(\tau)$ as baseband model is obtained from received and

the demodulated signals $\hat{\mathbf{b}}_k(t|\tau)$. According to the selection of the transmitted signatures, the complex valued entries of the transfer matrix are obtained as:

$$\hat{\mathbf{G}}_k(\tau) = \begin{bmatrix} \hat{g}_k^{HH}(\tau) & \hat{g}_k^{VH}(\tau) \\ \hat{g}_k^{HV}(\tau) & \hat{g}_k^{VV}(\tau) \end{bmatrix} = \int \begin{bmatrix} \hat{b}_k^H(\xi|\tau)a_k^H(\xi) & \hat{b}_k^H(\xi|\tau)a_k^V(\xi) \\ \hat{b}_k^V(\xi|\tau)a_k^H(\xi) & \hat{b}_k^V(\xi|\tau)a_k^V(\xi) \end{bmatrix} d\xi \quad (10)$$

The quadri-correlator 50 shown in figure 5B can be used to compute the elements (each HH, HV, VH, VV pair) of the matrix of expression (10).

Moreover, the software of diagnostic method includes the analysis of the transfer characteristic at the scattering nulls of the elements of $\hat{\mathbf{G}}_k(\tau)$ that accounts for the polarimetric information that can be derived from any of the known polarimetric transformation and decomposition from the scattering matrix $\hat{\mathbf{G}}_k(\tau)$.

The characteristics of the equivalent linear system represented by the scattering matrix $\hat{\mathbf{G}}_k(\tau)$ can be expressed for each entry (not exclusively) by: attenuation $\alpha_i$, delay of the response $\tau_i$, poles $p_i$ and damped oscillations $\omega_i$.

It is observed that the medium in which the backscattering field propagates contains an unknown mixture of fat, muscle, glands, duct tissues, etc... embedded in a fairly random heterogeneous structure, this prevents any simple interpretation from the complex backscattering energy, and this backscattering energy is fairly smoothly changing across space if compared to the same structure with malignant tumor. The indicator of parameters' smoothness accounts for the deviation of the characteristics of the equivalent linear system, for example the attenuation set $\{\alpha_1,\alpha_2,...,\alpha_N\}$ with respect to the mean, or median, for a subset related to neighbouring antennas with same polarization. Similar diagnostic indicators can be considered for a subset of frequencies, and/or polarizations, and/or any combination as indicated below.

In search for scattering nulls, these polarimetric transformations help the diagnostic indicator to highlight relevant interference effects in any polarization angle that yields multiple backscattering nulls at the specific positions of the probe antenna device 100 with respect to the antennas of the receiving device 200, frequency and polarization state of the probe antenna device 100 and the receiving device 200.

These transformations are related to the analysis of the polarization state of the scattering matrix $\hat{\mathbf{G}}_k(\tau)$ with the main aim to simplify the use of the diagnostic device as it minimizes the request for the clinician to rotate the probe antenna device 100 to match the null scattering in polarization (in some anatomic locations these rotations could be limited to few degrees such as in perineum, if not impossible). To better illustrate the detection method the preliminary analysis of the polarization state from polarimetric transformations and decomposition can be neglected in the following, since the polarimetric transformations and decomposition are clear to the skilled person.

It is also observed that elements of transfer matrix are time-varying because of radiation. When the probe antenna device 100 is placed near tissues (and is kept still) the transfer matrix elements changes vs. time $\tau$ with different behavior depending on the nature of the tissue and the total power. In case of healthy tissues, the scattered field is heterogeneous with sparse nulls while in presence of malignant tissues and calcifications there are complex interference patterns with deep nulls.

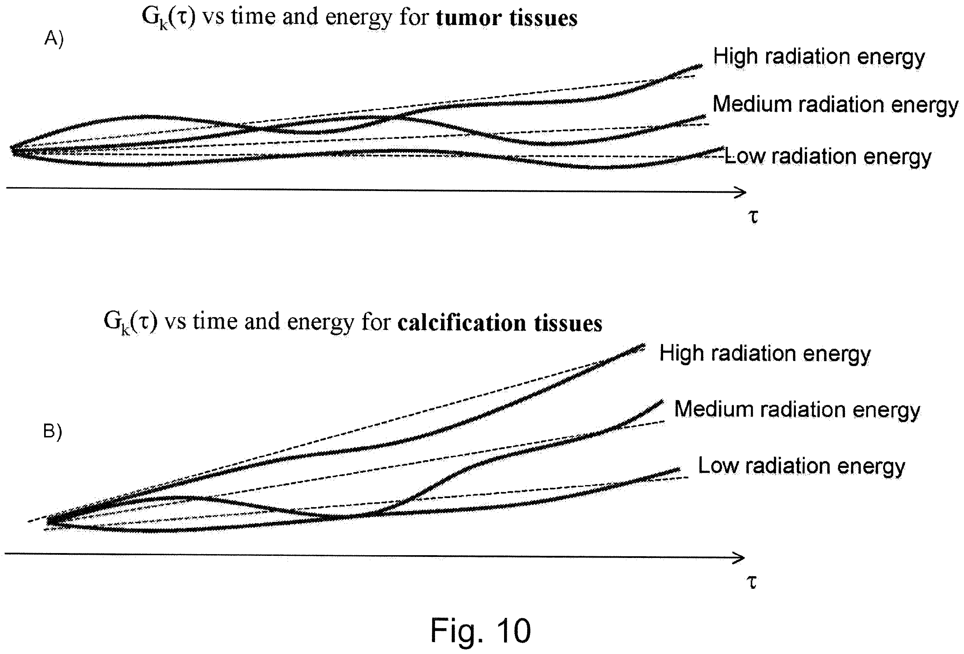

Particularly, once one (or more) antenna $R_{H1}$-$R_{VN}$ of the receiving device 200 is aligned with one null when the probe antenna device 100 is kept still, the scattering power (or transfer matrix gains) increases differently for tumors and calcifications. Figure 10A shows the behavior of the scattering power tumor tissues while Figure 10B refers to calcification tissues. Namely, in presence of tumor the scattering power at the null increases more slowly than for the calcification. The rate of increase of scattered power is used, according to a particular embodiment, to discriminate tissues depending on the response with respect to radiation power radiated by the probe antenna device 100.

It is observed that in case of a plurality of receiving antenna $R_{H1}$-$R_{VN}$, as for an array of antennas included in the probe antenna device 200, the aforementioned processing is simply duplicated for each Transmission/Reception link. The estimate of the transfer matrix for any link is $\hat{\mathbf{G}}_k(\tau,\ell)$ and polarimetric transformation is applied to every scattering matrix $\hat{\mathbf{G}}_k(\tau,\ell)$. The detection method includes an estimation of the set $\{\hat{\mathbf{G}}_k(\tau = q \times T, \ell)\}_{k=1,\ell=1,q=1}^{K,N,Q}$ from the ensemble of Q copies of received signals (over time $\mathbf{r}(t|\tau=T,\ell),\mathbf{r}(t|\tau=2T,\ell),...,\mathbf{r}(t|\tau=QT,\ell)$) over N antennas (index $\ell$) that contain the combination of multiple carriers (index k) and polarization. The detection method for N=1, and/or L=1 and/or one polarization is also accounted as special case.

Particularly, in addition to the estimation of the gain, the slope for each of the baseband filter can be also estimated within every measuring period T to locally approximate (for $qT \leq \tau < (q+1)T$ or equivalently $0 \leq \delta\tau < T$) the amplitude (or power) of the time-varying elements of the scattering matrix:

$$|\hat{\mathbf{G}}_k(qT+\delta\tau,\ell)|\approx \underbrace{\begin{bmatrix} \alpha_k^{HH}(qT,\ell) & \alpha_k^{VH}(qT,\ell) \\ \alpha_k^{HV}(qT,\ell) & \alpha_k^{VV}(qT,\ell) \end{bmatrix}}_{|\hat{\mathbf{G}}_k(qT,\ell)|} + \underbrace{\begin{bmatrix} \beta_k^{HH}(qT,\ell) & \beta_k^{VH}(qT,\ell) \\ \beta_k^{HV}(qT,\ell) & \beta_k^{VV}(qT,\ell) \end{bmatrix}}_{\boldsymbol{\delta}\hat{\mathbf{G}}_k(qT,\ell)} \times \delta\tau \quad (11)$$

The slopes indicated by variables β can be derived from the amplitude (or power) of the estimated matrix $\hat{\mathbf{G}}_k(\tau=qT,\ell)$ over neighboring periods with standard analysis or it can be derived from received signals over each frequency band if using flip-flop signatures as illustrated in Figure 9B.

As an example, an aggregate diagnostic indicator selects the set of minima values of elements of $|\hat{\boldsymbol{G}}_K(\tau=qT,\ell)|$ below a predefined amplitude threshold $\eta_\alpha$ and counts (operator count[.]) their occurrence out of the total set of 4QNK measurements as

$$p_a = \left\{ \begin{array}{l} \mathrm{count}\left[\{\alpha_k^{HH}(qT,\ell) < \eta_\alpha\}_{k=1,\ell=1,q=1}^{K,N,Q}\right] + \mathrm{count}\left[\{\alpha_k^{HV}(qT,\ell) < \eta_\alpha\}_{k=1,\ell=1,q=1}^{K,N,Q}\right] + \\ \mathrm{count}\left[\{\alpha_k^{VH}(qT,\ell) < \eta_\alpha\}_{k=1,\ell=1,q=1}^{K,N,Q}\right] + \mathrm{count}\left[\{\alpha_k^{VV}(qT,\ell) < \eta_\alpha\}_{k=1,\ell=1,q=1}^{K,N,Q}\right] \end{array} \right\} \Big/ 4QNK \quad (12)$$

$$\beta_a = \frac{4QNK}{p_a}\left\{ \sum_{\substack{k=1,\ell=1,q=1 \\ s.t.\alpha_k^{HH}(qT,\ell)<\eta_\alpha}}^{K,N,Q} \beta_k^{HH}(qT,\ell) + \sum_{\substack{k=1,\ell=1,q=1 \\ s.t.\alpha_k^{HV}(qT,\ell)<\eta_\alpha}}^{K,N,Q} \beta_k^{HV}(qT,\ell) + \sum_{\substack{k=1,\ell=1,q=1 \\ s.t.\alpha_k^{VH}(qT,\ell)<\eta_\alpha}}^{K,N,Q} \beta_k^{VH}(qT,\ell) + \sum_{\substack{k=1,\ell=1,q=1 \\ s.t.\alpha_k^{VV}(qT,\ell)<\eta_\alpha}}^{K,N,Q} \beta_k^{VV}(qT,\ell) \right\} \quad (13)$$

The measurement $p_a$ is a diagnostic parameter for the tumor or calcification as it counts the backscattering minima over the array of the receiving antennas included into the antenna device 200 as indicator of the complexity of the backscattering wave-field. The parameter $\beta_a$ models the time variation of the backscattering minima using a linear regression and it is:

$$|\beta_a| < \eta_\beta \quad \text{for tumor,} \quad (14)$$

$$|\beta_a| > \eta_\beta \quad \text{for calcification.} \quad (15)$$

Where the threshold $\eta_\beta$ is properly chosen.

**Experimental results**

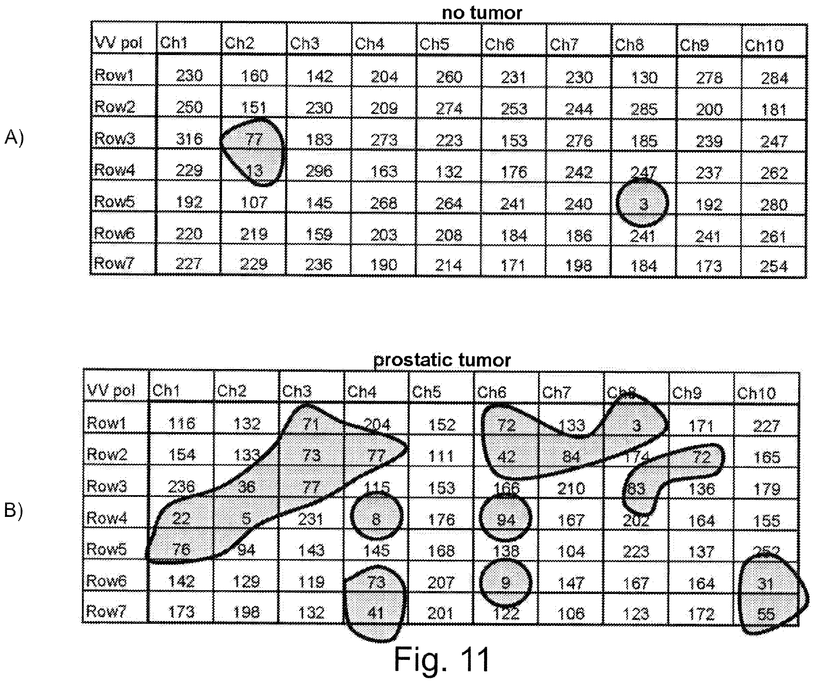

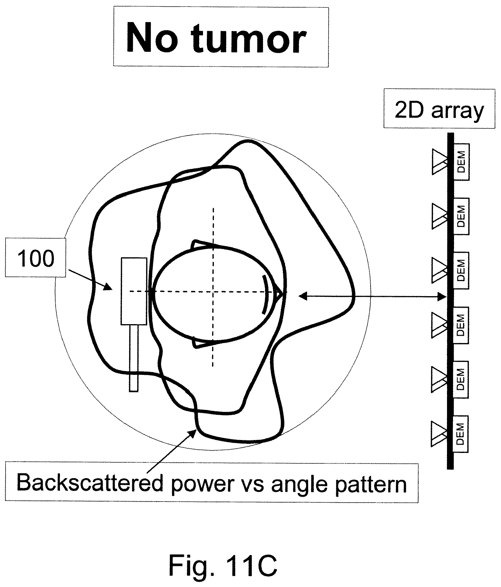

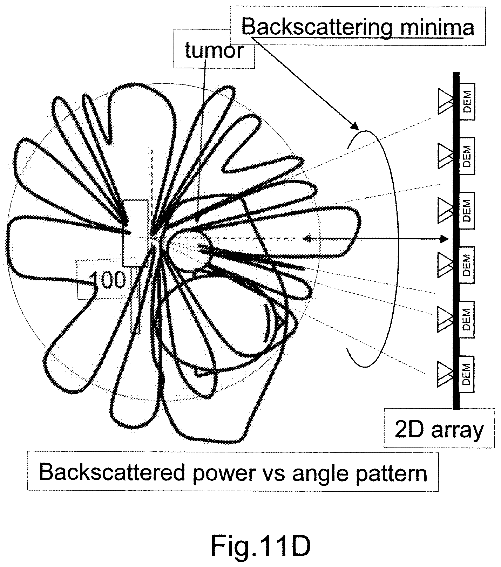

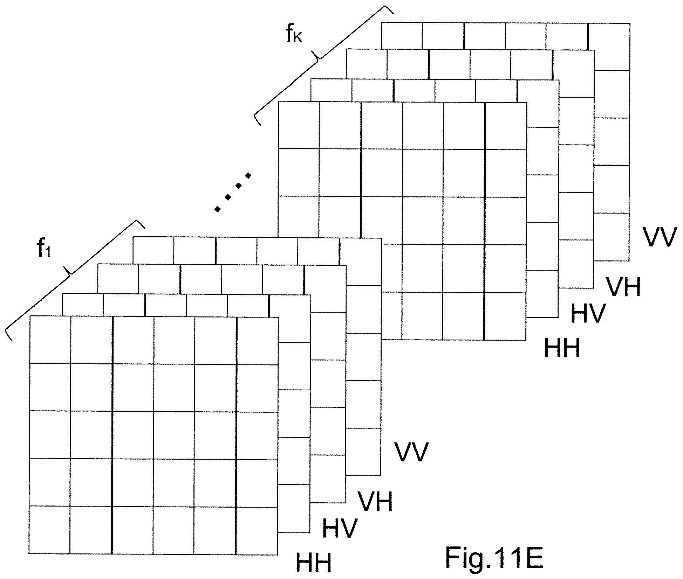

Figure 11 shows experimental results, under the for of tables, obtained using the detection system 1000 employing a probe antenna device 100 having two antennas (dual polarization) and a receiving device 200 having a 10X7 plane array of receiving antennas. By illustrating only one VV polarization Figure 11A shows the scattering energy in arbitrary units for prostate analysis without tumor while Figure 11B refers to the presence of tumor as clear from the multiple minima shown by the scattering energy distribution.

It is observed that the use of a probe antenna device 100 having more than one transmitting antenna and of a receiving device 200 having more than one receiving antenna is supportive to reduce the time of detection as the clinician is not forced to search for the spatial alignment with angular position of one of the scattering nulls when using only one receiving antenna $R_{H1}$ or $R_{V1}$ fixed in space.

Since the way body change the polarization state of the EM field is fairly complex to be modelled, transmitting antennas might have one or dual polarizations and the scattering field is measured either with one or dual polarizations. Once more, single polarization would imply that the clinician that holds the probe has to rotate it in search for the angle that minimizes the measured field, polarization analysis (both in transmission and in reception) reduces the time of the detection and the false-negative rate. Figure 11C and 11D show examples of power scattering patterns associated with a no-tumor condition (as in figure 11A) and tumor detection (as in figure 11B), respectively.

The scattering energy as represented in Figure 11A and 11B, by means of tables, are examples of results which can be displayed by the display 6 to be watched by the user of the detection system 1000. The relevant parameters (diagnostic indicators) resulting from the analysis made can be displayed also in accordance with other representation forms (e.g. a list of parameters). Particularly, a plurality of scattering energy tables (figure 11E) associated with a position of the antenna probe device 100, each employed FDM frequency and each pair of used polarizations can be displayed by the display 6.

Moreover, it is observed that teaching of the present invention provides a diagnostic/detection tool for early-detection mainly, but not exclusively, for those tumors that are located close to the skin surface (such as prostate, colon-rectum, breast) based on the interfering effect between a radiating antenna operating in the range of 350-600MHz placed at skin contact in close EM coupling with malignant tissues (for detection of prostate cancer the antenna is placed over the perineum within 3-4 cm from prostate).

**Software architecture scheme**

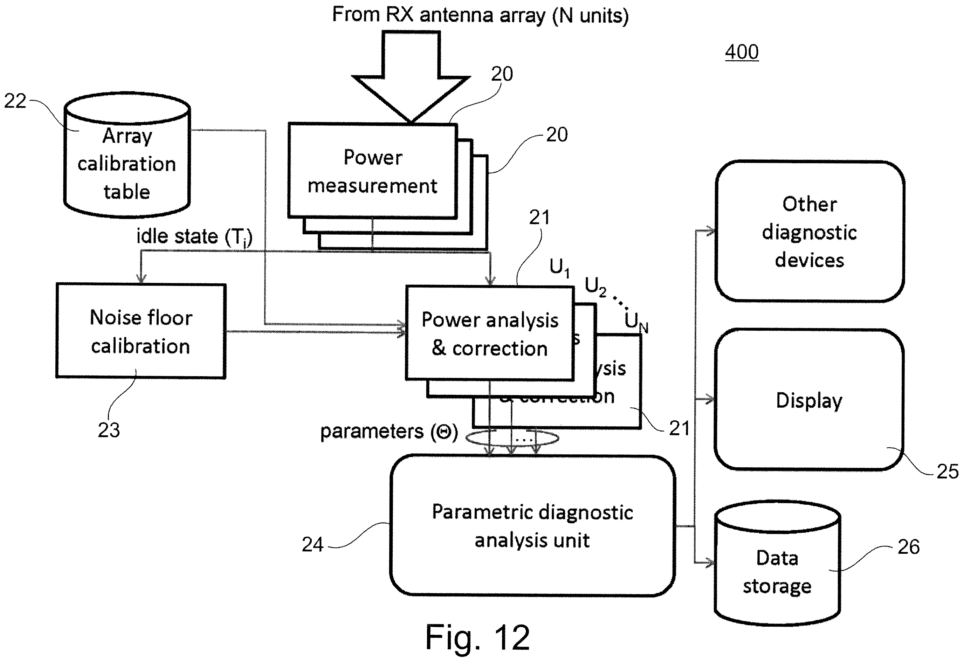

Figure 12 shows by means of a schematization an example of a possible architecture of a software 400 employable by the processing unit 300 to carry out the described methods. Software 400 comprises a plurality of measurement software modules 20 which are configured to suitably process the signals received by receiving device 200 to measure for example the received power and so acting as an envelope detector. Moreover, the software 400 includes a plurality of power analysis and correction modules 21 which allows to provide a plurality of parameters (such as attenuation $\alpha_i$, delay of the response $\tau_i$, poles $p_i$ and damped oscillations $\omega_i$) representing the estimated matrix $\hat{\mathbf{G}}_k(\tau)$, according to equation (10).

Moreover, software 400 is provided with an array calibration database module 22 which allows suitably configuring, in a calibration step, the power analysis and correction modules 21. A noise floor calibration module 23 allows substantially compensating errors in the power analysis and correction modules 21 due to interferences produced by external objects or other interfering sources.

Software 400 is provided with a parametric diagnostic analysis module 24 which performs computing according to expressions (11), (12), (13), (14) and (15) in order to provide detection and diagnostic information, which can be visualized by a display module 25 of the display 6 and stored into a data base 26. Advantageously, an interface module 27 which interfaces the parametric diagnostic analysis module 24 with external diagnostic devices (e.g. an ultrasound device) can be provided.

As it is clear from the description of the embodiment of Figure 1 and the operating method thereof, the detection system 1000 allows obtaining a reliable early-detection of suspicious malignant tumors, that is paired with a method for processing the backscattering data, without the need of specialized expertise in interpretation of body-images.

Since therapies can reduce the anomalous electric contrasts of tumor tissues, the detection system 1000 can be also used as a tool for early-evaluation of the efficacy of any of the therapies such as chemotherapy while these therapies are in progress rather than from the investigation of the reduction of tumor size as by inner body imaging devices such as CAT and/or MRI.

It is also underlined that the use of a probe antenna device 100 employing dual-polarization antennas minimizes the need for the clinician to rotate the portable probe device 100 when operating in search for tumors and/or calcifications.

The system and method described can detect the presence and localize anomalies with a minimum amount of time for large-scale screening programs.

Moreover, the embodiment showing the capability to separate malignant tissues from calcifications by evaluating and characterizing the time-varying response of the scattering pattern from the specific analysis of the scattering nulls, or minima, is particularly advantageous.

It is also noticed that, as an example, the described detection system and method can use for diagnostic purposes the tissues absorption at low frequency (below 1GHz) even if the wavelength (typically larger than 3-4cm when propagating in fat tissue) is much larger than the size of malignant tumor (normally below 1-2 cm).

In this condition the physical phenomena used for diagnostic purposes is not translucency, as for microwave imaging using higher frequencies (above 1-2GHz with bandwidth larger than 1GHz for resolution issues). On the contrary and as already described, the involved phenomena is the near-field coupling that is used to infer the presence of malignant tumors, or other inhomogeneities such as calcifications that have anomalous electrical contrasts, from the nulls in the backscattered pattern.

It is observed that the detection system 1000 does not provide any direct image of the tumor or direct quantitative estimation of electrical properties of tissues as for inverse scattering, it rather detects the presence or not by scanning in different body-locations where malignant tumors are searched by probe-proximity.

Moreover, with reference to the probe antenna device 100, decoupling the incident signal generation from the radiating antenna portion 10 have the benefits of:

1) reducing the size of the radiating antenna portion 10 of the probe antenna device;
2) increasing the coupling between the antenna and tissues without the need to incorporate the investigated tissues as part of the signal and frequency generation;
3) control the frequency and the pattern of signal generation independently of the tissues to be coupled with;
4) optimize the design and synchronization of generated signatures for interference mitigation;
5) avoid or minimize the generation of multiple harmonics out of the fundamental carrier frequency for example in the range 350-550MHz.

**Claims**

1. A tissue anomaly detection system (1000), comprising:

   a probe antenna device (100) structured to radiate a narrowband incident radio-frequency signal at a frequency below 700 MHz to irradiate a tissue in near-field conditions so as to produce a resulting radio-frequency signal;
   a variable frequency oscillator (E-LS) that operates as an electromagnetic wave source connected to first and second modulating modules (MOD1-MOD2) to provide electromagnetic signals;
   a controller module (4) configured to control the a variable frequency oscillator (E-LS) that operates as electromagnetic wave source;
   a receiving device (200) structured to receive the resulting signal and provide corresponding received data;
   a processing module (300) structured to process the received data to provide an information indicating detection of an anomaly in said tissue;
   wherein:

   the probe antenna device has a fixed radiation frequency range and is structured so as that the resulting signal is a scattered signal resulting from a combination of the incident signal and an induced radio-frequency signal produced by the radiated tissue; the probe antenna device (100) includes at least one first transmitting antenna (1) connected to the first modulating module (MOD1) and structured to radiate first electromagnetic waves having a first linear polarization and at least one second transmitting antenna (2) connected to the second modulating module (MOD2) and structured to radiate second electromagnetic waves having a second linear polarization orthogonal to the first polarization;
   the receiving device (200) includes at least one receiving antenna unit including two receiving antennas ($R_{H1}$-$R_{V1}$) orientated to receive electromagnetic waves according to orthogonal linear polarisation and at least one demodulating module ($DEM_{11}$) ;
   the processing module (300) is structured to analyse from the resulting data an electrical field power scattering versus frequency pattern and detect said anomaly associated with nulls or minima in said pattern.

2. The detection system of claim 1, wherein said narrowband incident radio-frequency signal shows a bandwidth so that a response of an interaction of the narrowband incident radio-frequency signal with the tissue is constant within the bandwidth.

3. The system of claim 1, wherein said probe antenna device (100) includes a first portion (10) to be put in contact with a body and provided with said first transmitting antenna (1) and a support portion having (11) electromagnetic field absorbing material (12); the first transmitting antenna (1) is configured to generate a linearly polarized electromagnetic field.

4. The system of claim 1, wherein the receiving device (200) includes at least one first receiving antenna ($R_{H1}$) structured to receive said first electromagnetic waves and is connected to a demodulating module (DEM11).

5. The system of claim 4, wherein the receiving device (200) includes at least one second receiving antenna ($R_{V1}$) structured to receive said second electromagnetic waves and is connected to the demodulating module (DEM11).

6. The system of claim 1, wherein said probe antenna device (100) is adapted to radiate the incident radio-frequency signal comprising a plurality of carrier signals in accordance with a frequency division multiplexing technique and said receiving device is adapted to process the resulting signal according to an inverse frequency division multiplexing technique.

7. The system in accordance with at least claim 4, wherein:

the first and second modulating modules (MOD1-MOD2) are adapted to modulate an amplitude of a carrier signal according to a modulating signal to obtain a modulated incident signal to be radiated by the probe antenna device, and

said demodulating module ($DEM_{11}$) is structured to demodulate the resulting signal as received at the receiving device (200) and provide a received scattered signal.

8. The system of claim 5 and 7, wherein the modulating signal is a set of delayed square waveforms.

9. The system of claims 5 and 7, wherein the probe antenna device (100) is structured to radiate the incident signal by alternating the activation of the at least the first antenna (1) and the at least the second antenna (2).

10. The system of claim 7, wherein the processing module (300) is structured to process the receiving scattered signal on the basis of said modulating signal to compute a scattering matrix to be analysed to detect said nulls or minima.

11. The system of claim 10, wherein the processing module (300) is configured to analyse the scattering matrix and count said nulls or minima below a threshold associated with an anomaly.

12. The system of claim 11, wherein the processing module (300) is configured to analyse the scattering matrix to determining a time slope parameter associated with a time-variation of the backscattering behavior of the radiated anomaly.

13. The system of claim 12, wherein the processing module is structured to compare the time slope parameter with a reference value to determine if the anomaly is a tumor or calcification.

14. The system of claim 1, wherein the incident signal is a non-ionizing radio-frequency wave having a carrier frequency included in the range of 350-600 MHz.

15. The system of claim 1, wherein the probe antenna device is configured to generate radio-frequency signals at K frequencies $f_1, f_2,..., f_K$ chosen within a predefined frequency span around a central carrier frequency $f_0$ with 350MHz $<f_0<$ 550MHz; wherein the frequency span of the K frequencies is below 100 MHz.

16. A method, comprising the steps of:

providing a tissue anomaly detection system (1000) according to any preceding claim comprising a probe antenna device (100) having a fixed radiation frequency range; the probe antenna device (100) includes at least one first antenna (1) connected to a first modulating module (MOD1) and structured to radiate first electromagnetic waves having a first linear polarization and at least one second transmitting antenna (2) connected to a second modulating module (MOD2) and structured to radiate second electromagnetic waves having a second linear polarization orthogonal to the first polarization;

and a variable frequency oscillator that operates as an electromagnetic wave source (E-LS) connected to the first modulating module (MOD1) and the second modulating module (MOD2) to provide electromagnetic signals;

controlling by controller module (4) the variable frequency oscillator (E-LS) that operates as an electromagnetic wave source;

moving the probe antenna device (100) radiating an incident narrowband radio-frequency signal at a frequency below 700 MHz to irradiate a tissue in near-field conditions;

producing a scattered signal resulting from a combination of the incident signal and an induced radio-frequency signal produced by the irradiated tissue;

receiving at a receiving device (200) the scattered signal and provide corresponding received data representing an electrical field power scattering pattern; includes at least a receiving antenna unit including two receiving antennas ($R_{H1}$-$R_V$) and at least one demodulating module ($DEM_{11}$) ;

processing the received data to provide an information associated with power nulls or power minima of the electrical field power scattering pattern.

**Patentansprüche**

1. System zur Erfassung von Gewebeanomalien (1000), umfassend:

eine Sondenantenneneinrichtung (100), die strukturiert ist, um ein schmalbandiges, einfallendes Hochfrequenzsignal bei einer Frequenz unter 700 MHz auszustrahlen, um ein Gewebe in Nahfeldbedingungen zu bestrahlen, um so ein resultierendes Hochfrequenzsignal zu erzeugen;

einen Oszillator mit variabler Frequenz (E-LS), der als eine elektromagnetische Wellenquelle arbeitet, die mit einem ersten und einem zweiten Modulationsmodul (MOD1-MOD2) verbunden ist, um elektromagnetische Signale bereitzustellen;

ein Steuerungsmodul (4), das konfiguriert ist, um den Oszillator mit variabler Frequenz (E-LS) zu steuern, der als elektromagnetische Wellenquelle arbeitet;

eine Empfangseinrichtung (200), die strukturiert ist, um das resultierende Signal zu empfangen und entsprechende empfangene Daten bereitzustellen;

ein Verarbeitungsmodul (300), das strukturiert ist, um die empfangenen Daten zu verarbeiten, um eine Information bereitzustellen, die die Erfassung einer Anomalie in dem Gewebe anzeigt;

wobei:

die Sondenantenneneinrichtung einen festen Strahlungsfrequenzbereich aufweist und so strukturiert ist, dass das resultierende Signal ein gestreutes Signal ist, das aus einer Kombination des einfallenden Signals und eines induzierten Hochfreuenzsignals resultiert, das durch das abgestrahlte Gewebe erzeugt wird; wobei die Sondenantenneneinrichtung (100) mindestens eine erste Modulationsantenne (1), die mit dem ersten Modulationsmodul (MOD1) verbunden ist und strukturiert ist, um erste elektromagnetische Wellen abzustrahlen, die eine erste lineare Polarisation haben, und mindestens eine zweite Sendeantenne (2) einschließt, die mit dem zweiten Modulationsmodul (MOD2) verbunden ist und strukturiert ist, um zweite elektromagnetische Wellen abzustrahlen, die eine zweite lineare Polarisation haben, die orthogonal zu der ersten linearen Polarisation ist;

wobei die Empfangseinrichtung (200) mindestens eine Empfangsantenneneinheit, die zwei Empfangsantennen ($R_{H1}$-$R_{V1}$) einschließt, die so ausgerichtet sind, dass sie elektromagnetische Wellen gemäß der orthogonalen linearen Polarisation empfangen, und mindestens ein Demodulationsmodul ($DEM_{11}$) einschließt;

wobei das Verarbeitungsmodul (300) strukturiert ist, um aus den resultierenden Daten eine Streuung der elektrischen Feldleistung gegenüber einem Frequenzmuster zu analysieren und die Anomalie zu erfassen, die Nullstellen oder Minima in dem Muster zugeordnet ist.

2. System nach Anspruch 1, wobei das schmalbandige, einfallende Hochfrequenzsignal eine Bandbreite aufweist, so dass eine Reaktion auf eine Interaktion des schmalbandigen, einfallenden Hochfrequenzsignals mit dem Gewebe innerhalb der Bandbreite konstant ist.

3. System nach Anspruch 1, wobei die Sondenantenne (100) einen ersten Abschnitt (10) einschließt, der mit einem Körper in Kontakt gebracht werden soll und mit der ersten Sendeantenne (1) und mit einem Stützabschnitt (11) versehen ist, der ein Material zur elektromagnetischen Feldabsorption (12) hat; wobei die erste Sendeantenne (1) konfiguriert ist, um ein lineares polarisiertes elektromagnetisches Feld zu erzeugen.

4. System nach Anspruch 1, wobei die Empfangseinrichtung (200) mindestens eine erste Empfangsantenne ($R_{H1}$) einschließt, die strukturiert ist, um die ersten elektromagnetischen Wellen zu empfangen und mit einem Demodulationsmodul (DEM11) verbunden ist.

5. System nach Anspruch 4, wobei die Empfangseinrichtung (200) mindestens eine zweite Empfangsantenne ($R_{V1}$) einschließt, die strukturiert ist, um die zweiten elektromagnetischen Wellen zu empfangen, und mit dem Demodulationsmodul (DEM11) verbunden ist.

6. System nach Anspruch 1, wobei die Sondenantenneneinrichtung (100) eingerichtet ist, um das einfallende Hochfrequenzsignal, das mehrere Trägersignale umfasst, gemäß einer Frequenzmultiplextechnik abzustrahlen, und wobei die Empfangseinrichtung eingerichtet ist, das resultierende Signal gemäß einer inversen Frequenzmultiplextechnik zu verarbeiten.

7. System mindestens gemäß Anspruch 4, wobei:

das erste und das zweite Modulationsmodul (MOD1-MOD2) eingerichtet sind, um eine Amplitude eines Trägersignals gemäß einem Modulationssignal zu modulieren, um ein moduliertes, einfallendes Signal zu erhalten, das von der Sondenantenneneinrichtung abgestrahlt werden soll, und

wobei das Demodulationsmodul (DEM$_{11}$) strukturiert ist, das resultierende Signal, wie es bei der Empfangseinrichtung (200) empfangen wird, zu demodulieren und ein empfangenes gestreutes Signal bereitzustellen.

8. System nach Anspruch 5 und 7, wobei das Modulationssignal ein Satz von verzögerten rechteckigen Wellenformen ist.

9. System nach den Ansprüchen 5 und 7, wobei die Sondenantenneneinrichtung (100) strukturiert ist, um das einfallende Signal durch Wechseln der Aktivierung der mindestens ersten Antenne (1) und der mindestens zweiten Antenne (2) abzustrahlen.

10. System nach Anspruch 7, wobei das Verarbeitungsmodul (300) strukturiert ist, um das empfangene gestreute Signal auf der Basis des Modulationssignals zu verarbeiten, um eine Streumatrix zu berechnen, die analysiert werden soll, um die Nullpunkte oder Minima zu erfassen.

11. System nach Anspruch 10, wobei das Verarbeitungsmodul (300) konfiguriert ist, um die Streuungsmatrix zu analysieren, und die Nullstellen oder Minima unterhalb einer Schwelle zu zählen, die einer Anomalie zugeordnet sind.

12. System nach Anspruch 11, wobei das Verarbeitungsmodul (300) konfiguriert ist, um die Streumatrix zu analysieren, um einen Zeitsteigungsparameter zu bestimmen, der einer Zeitvariation des Rückstreuverhaltens der bestrahlten Anomalie zugeordnet ist.

13. System nach Anspruch 12, wobei das Verarbeitungsmodul strukturiert ist, um den Zeitsteigungsparameter mit einem Referenzwert zu vergleichen, um zu bestimmen, ob die Anomalie ein Tumor oder eine Verkalkung ist.

14. System nach Anspruch 1, wobei das einfallende Signal eine nicht ionisierende Hochfrequenzwelle ist, die eine Trägerfrequenz hat, die im Bereich von 350 - 600 MHz eingeschlossen ist.

15. System nach Anspruch 1, wobei die Sondenantenneneinrichtung konfiguriert ist, um Hochfrequenzsignale mit K Frequenzen $f_1$, $f_2$, ... $f_k$ zu erzeugen, die innerhalb einer vordefinierten Frequenzspanne um eine zentrale Trägerfrequenz fo mit 350 MHz < fo < 550 MHz ausgewählt sind; wobei die Frequenzspanne der K Frequenzen unter 100 MHz liegt.

16. Verfahren, das die Schritte umfasst:

Bereitstellen eines Systems zur Erfassung von Gewebeanomalien (1000) nach irgendeinem vorhergehenden Anspruch, das umfasst:

eine Sondenantenneneinrichtung (100), die einen festen Strahlungsfrequenzbereich hat; wobei die Sondenantenneneinrichtung (100) mindestens eine erste Antenne (1), die mit einem ersten Modulationsmodul (MOD1) verbunden ist und strukturiert ist, um erste elektromagnetische Wellen, die eine erste lineare Polarisation haben, abzustrahlen, und mindestens eine zweite Sendeantenne (2) einschließt, die mit einem zweiten Modulationsmodul (MOD2) verbunden ist und strukturiert ist, um zweite elektromagnetische Wellen, die eine zweite lineare Polarisation haben. abzustrahlen, die orthogonal zu der ersten Polarisation ist; und einen Oszillator mit variabler Frequenz, der als eine elektromagnetische Wellenquelle (E-LS) arbeitet, die mit dem ersten Modulationsmodul (MOD1) und dem zweiten Modulationsmodul (MOD2) verbunden ist, um elektromagnetische Signale bereitzustellen;
Steuern des Oszillators (E-LS) mit variabler Frequenz , der als eine elektromagnetische Wellenquelle arbeitet, durch das Steuermodul (4);
Bewegen der Sondenantenneneinrichtung (100), die ein schmalbandiges einfallendes Hochfrequenzsignal mit einer Frequenz unter 700 MHz abstrahlt, um ein Gewebe unter Nahfeldbedingungen zu bestrahlen;
Erzeugen eines gestreuten Signals, das aus einer Kombination des einfallenden Signals und eines induzierten Hochfrequenzsignals resultiert, das durch das bestrahlte Gewebe erzeugt wird;
Empfangen des gestreuten Signals bei einer Empfangseinrichtung (200) und Bereitstellen entsprechender empfangener Daten, die ein Streuungsmuster der elektrischen Feldleistung darstellen; wobei sie mindestens eine Empfangsantenneneinheit mit zwei Empfangsantennen (R$_{H1}$-R$_v$) und mindestens einem Demodulationsmodul (DEM11) einschließt;
Verarbeiten der empfangenen Daten, um eine Information bereitzustellen, die den Leistungsnullstellen oder Leistungsminima des Streuungsmusters des elektrischen Feldes zugeordnet ist.

# EP 2 465 428 B1

## Revendications

1. Système de détection d'anomalie de tissu (1000), comprenant :

   un dispositif d'antenne de sonde (100) structuré pour rayonner un signal de radiofréquence incident à bande étroite à une fréquence en dessous de 700 MHz pour irradier un tissu dans des conditions de champ proche de façon à produire un signal de radiofréquence résultant ;
   un oscillateur à fréquence variable (E-LS) qui fonctionne en tant que source d'ondes électromagnétiques raccordé à des premier et second modules de modulation (MOD1-MOD2) pour fournir des signaux électromagnétiques ;
   un module de commande (4) configuré pour commander l'oscillateur à fréquence variable (E-LS) qui fonctionne en tant que source d'ondes électromagnétiques ;
   un dispositif de réception (200) structuré pour recevoir le signal résultant et fournir des données reçues correspondantes ;
   un module de traitement (300) structuré pour traiter les données reçues pour fournir une information indiquant la détection d'une anomalie dans ledit tissu ;
   dans lequel :

   le dispositif d'antenne de sonde a une plage de fréquences de rayonnement fixe et est structuré de sorte que le signal résultant soit un signal diffus résultant d'une combinaison du signal incident et d'un signal de radiofréquence induit produit par le tissu rayonné ; le dispositif d'antenne de sonde (100) comporte au moins une première antenne d'émission (1) raccordée au premier module de modulation (MOD1) et structurée pour rayonner des premières ondes électromagnétiques ayant une première polarisation linéaire et au moins une seconde antenne d'émission (2) raccordée au second module de modulation (MOD2) et structurée pour rayonner des secondes ondes électromagnétiques ayant une seconde polarisation linéaire orthogonale à la première polarisation ;
   le dispositif de réception (200) comporte au moins une unité d'antennes de réception comportant deux antennes de réception ($R_{H1}$-$R_{V1}$) orientées pour recevoir des ondes électromagnétiques selon la polarisation linéaire orthogonale et au moins un module de démodulation ($DEM_{11}$) ;
   le module de traitement (300) est structuré pour analyser à partir des données résultantes une diffusion de puissance de champ électrique en fonction d'un schéma de fréquence et détecter ladite anomalie associée à des valeurs nulles ou des minima dans ledit schéma.

2. Système de détection selon la revendication 1, dans lequel ledit signal de radiofréquence incident à bande étroite montre une largeur de bande de sorte qu'une réponse d'une interaction du signal de radiofréquence incident à bande étroite avec le tissu soit constante au sein de la largeur de bande.

3. Système selon la revendication 1, dans lequel ledit dispositif d'antenne de sonde (100) comporte une première portion (10) à mettre en contact avec un corps et dotée de ladite première antenne d'émission (1) et une portion de support ayant (11) un matériau d'absorption de champ électromagnétique (12) ; la première antenne d'émission (1) est configurée pour générer un champ électromagnétique polarisé linéairement.

4. Système selon la revendication 1, dans lequel le dispositif de réception (200) comporte au moins une première antenne de réception ($R_{H1}$) structurée pour recevoir lesdites premières ondes électromagnétiques et est raccordé à un module de démodulation (DEM11).

5. Système selon la revendication 4, dans lequel le dispositif de réception (200) comporte au moins une seconde antenne de réception ($R_{V1}$) structurée pour recevoir lesdites secondes ondes électromagnétiques et est raccordé au module de démodulation (DEM11).

6. Système selon la revendication 1, dans lequel ledit dispositif d'antenne de sonde (100) est adapté pour rayonner le signal de radiofréquence incident comprenant une pluralité de signaux de porteuse conformément à une technique de multiplexage par répartition en fréquence et ledit dispositif de réception est adapté pour traiter le signal résultant selon une technique de multiplexage par répartition en fréquence inverse.

7. Système selon au moins la revendication 4, dans lequel :

   les premier et second modules de modulation (MOD1-MOD2) sont adaptés pour moduler une amplitude d'un signal de porteuse selon un signal de modulation pour obtenir un signal incident modulé à rayonner par le dispositif d'antenne de sonde, et
ledit module de démodulation (DEM$_{11}$) est structuré pour démoduler le signal résultant tel qu'il est reçu au niveau du dispositif de réception (200) et fournir un signal diffus reçu.

**8.** Système selon les revendications 5 et 7, dans lequel le signal de modulation est un ensemble de formes d'ondes carrées retardées.

**9.** Système selon les revendications 5 et 7, dans lequel le dispositif d'antenne de sonde (100) est structuré pour rayonner le signal incident en alternant l'activation de l'au moins la première antenne (1) et de l'au moins la seconde antenne(2).

**10.** Système selon la revendication 7, dans lequel le module de traitement (300) est structuré pour traiter le signal diffus de réception sur la base dudit signal de modulation pour calculer une matrice de diffusion à analyser pour détecter lesdites valeurs nulles ou lesdits minima.

**11.** Système selon la revendication 10, dans lequel le module de traitement (300) est configuré pour analyser la matrice de diffusion et compter lesdites valeurs nulles ou lesdits minima en dessous d'un seuil associé à une anomalie.

**12.** Système selon la revendication 11, dans lequel le module de traitement (300) est configuré pour analyser la matrice de diffusion pour déterminer un paramètre de pente de temps associé à une variation de temps du comportement de rétrodiffusion de l'anomalie rayonnée.

**13.** Système selon la revendication 12, dans lequel le module de traitement est structuré pour comparer le paramètre de pente de temps avec une valeur de référence pour déterminer si l'anomalie est une tumeur ou une calcification.

**14.** Système selon la revendication 1, dans lequel le signal incident est une onde radiofréquence non ionisante ayant une fréquence porteuse incluse dans la plage de 350 - 600 MHz.

**15.** Système selon la revendication 1, dans lequel le dispositif d'antenne de sonde est configuré pour générer des signaux de radiofréquence à K fréquences $f_1$, $f_2$, ..., $f_K$ choisies dans un étalement de fréquences prédéfini autour d'une fréquence porteuse centrale $f_0$ avec 350 MHz < $f_0$ < 550 MHz ; dans lequel l'étalement de fréquences des K fréquences est en dessous de 100 MHz.

**16.** Procédé, comprenant les étapes de :

fourniture d'un système de détection d'anomalie de tissu (1000) selon l'une quelconque des revendications précédentes comprenant un dispositif d'antenne de sonde (100) ayant une plage de fréquences de rayonnement fixe ; le dispositif d'antenne de sonde (100) comporte au moins une première antenne (1) raccordée à un premier module de modulation (MOD1) et structurée pour rayonner des premières ondes électromagnétiques ayant une première polarisation linéaire et au moins une seconde antenne d'émission (2) raccordée à un second module de modulation (MOD2) et structurée pour rayonner des secondes ondes électromagnétiques ayant une seconde polarisation linéaire orthogonale à la première polarisation ; et
un oscillateur à fréquence variable qui fonctionne en tant que source d'ondes électromagnétiques (E-LS) raccordé au premier module de modulation (MOD1) et au second module de modulation (MOD2) pour fournir des signaux électromagnétiques ;
la commande par un module de commande (4) de l'oscillateur à fréquence variable (E-LS) qui fonctionne en tant que source d'ondes électromagnétiques ;
le déplacement du dispositif d'antenne de sonde (100) rayonnant un signal de radiofréquence à bande étroite incident à une fréquence en dessous de 700 MHz pour irradier un tissu dans des conditions de champ proche ;
la production d'un signal diffus résultant d'une combinaison du signal incident et d'un signal de radiofréquence induit produit par le tissu irradié ;
la réception au niveau d'un dispositif de réception (200) du signal diffus et la fourniture de données reçues correspondantes représentant un schéma de diffusion de puissance de champ électrique ; comporte au moins une unité d'antennes de réception comportant deux antennes de réception ($R_{H1}$-$R_V$) et au moins un module de démodulation (DEM$_{11}$) ;
le traitement des données reçues pour fournir une information associée à des valeurs nulles de puissance ou des minima de puissance du schéma de diffusion de puissance de champ électrique.

Fig. 1

EP 2 465 428 B1

Fig. 2

$$\alpha s(t) e^{j\frac{2\pi\sqrt{\varepsilon}}{\lambda}\Delta}$$

Fig. 4

Fig.3

Fig. 5A

EP 2 465 428 B1

Fig. 5B

Fig. 6

Fig. 7

Spectral view of generated H or V signal

A)

$f_1$ $f_2$ $f_3$ $\cdots$ $f_k$

Spectral view of received H or V signal (after H/V decoupling)

$g_1(\tau)$

$g_2(\tau)$

scattering null

$g_3(\tau)$

$g_k(\tau)$

B)

$f_1$ $f_2$ $f_3$ $\cdots$ $f_k$

Fig. 8

Fig. 9

A) $G_k(\tau)$ vs time and energy for **tumor tissues**

High radiation energy

Medium radiation energy

Low radiation energy

$\tau$

B) $G_k(\tau)$ vs time and energy for **calcification tissues**

High radiation energy

Medium radiation energy

Low radiation energy

$\tau$

Fig. 10

**no tumor**

| VV pol | Ch1 | Ch2 | Ch3 | Ch4 | Ch5 | Ch6 | Ch7 | Ch8 | Ch9 | Ch10 |
|---|---|---|---|---|---|---|---|---|---|---|
| Row1 | 230 | 160 | 142 | 204 | 260 | 231 | 230 | 130 | 278 | 284 |
| Row2 | 250 | 151 | 230 | 209 | 274 | 253 | 244 | 285 | 200 | 181 |
| Row3 | 316 | 77 | 183 | 273 | 223 | 153 | 276 | 185 | 239 | 247 |
| Row4 | 229 | 13 | 296 | 163 | 132 | 176 | 242 | 247 | 237 | 262 |
| Row5 | 192 | 107 | 145 | 268 | 264 | 241 | 240 | 3 | 192 | 280 |
| Row6 | 220 | 219 | 159 | 203 | 208 | 184 | 186 | 241 | 241 | 261 |
| Row7 | 227 | 229 | 236 | 190 | 214 | 171 | 198 | 184 | 173 | 254 |

A)

**prostatic tumor**

| VV pol | Ch1 | Ch2 | Ch3 | Ch4 | Ch5 | Ch6 | Ch7 | Ch8 | Ch9 | Ch10 |
|---|---|---|---|---|---|---|---|---|---|---|
| Row1 | 116 | 132 | 71 | 204 | 152 | 72 | 133 | 3 | 171 | 227 |
| Row2 | 154 | 133 | 73 | 77 | 111 | 42 | 84 | 174 | 72 | 165 |
| Row3 | 236 | 36 | 77 | 115 | 153 | 166 | 210 | 83 | 136 | 179 |
| Row4 | 22 | 5 | 231 | 8 | 176 | 94 | 167 | 202 | 164 | 155 |
| Row5 | 76 | 94 | 143 | 145 | 168 | 138 | 104 | 223 | 137 | 252 |
| Row6 | 142 | 129 | 119 | 73 | 207 | 9 | 147 | 167 | 164 | 31 |
| Row7 | 173 | 198 | 132 | 41 | 201 | 122 | 106 | 123 | 172 | 55 |

B)

Fig. 11

**No tumor**

2D array

100

Backscattered power vs angle pattern

Fig. 11C

EP 2 465 428 B1

Fig.11D

Fig.11E

400

From RX antenna array (N units)

Power measurement 20

Array calibration table 22

idle state ($T_i$)

Noise floor calibration 23

Power analysis & correction 21

$U_1$ $U_2$ ... $U_N$

parameters ($\Theta$)

Parametric diagnostic analysis unit 24

Other diagnostic devices

Display 25

Data storage 26

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6061589 A
- EP 1264576 A
- US 20090129652 A
- WO 200107909 A

### Non-patent literature cited in the description

- **A.JEMAL ; R.SIEGEL ; J.XU ; E.WARD.** Cancer Statistics - 2010. *Cancer J Clin.,* September 2010, vol. 60, 277-300
- Dielectric properties of tissues. **K.R.FOSTER ; H.P.SCHWAN.** Handbook of Biological Effects of Electromagnetic Field. CRC Press, 1996
- **S. GABRIEL ; R. W. LAU ; C. GABRIEL.** The dielectric properties of biological tissues: III. Parametric models for the dielectric spectrum of tissues. *Phys. Med., Biol.,* 1996, vol. 41 (11), 2271-2293
- **K.R.FOSTER ; H.P. SCHWAN.** Dielectric properties of tissues and biological materials: a critical review. *Crit. Rev. Biomed. Eng.,* 1989, vol. 17, 25-104
- **P.VAUPEL ; F. KALLINOWSKI ; P.OKUNIEFF.** Blood flow, oxygen and nutrient supply, and metabolic microenvironment of human tumors: a review. *Cancer Res.,* December 1989, vol. 49, 6449-6465
- **J.S.MICHAELSON et al.** Predicting the survival of patients with breast carcinoma using tumor size. *Cancer,* August 2002, vol. 95 (4), 713-723
- **E.C.FEAR ; S.H.HAGNESS ; P. M. MEANEY ; M. OKONIEWSKI ; M. A.STUCHLY.** Enhancing breast tumor detection with near field imaging. *IEEE Microwave Mag.,* March 2002, vol. 3, 8-56
- **S. C. HAGNESS ; A. TAFLOVE ; J. E. BRIDGES.** Two-dimensional FDTD analysis of a pulsed microwave confocal system for breast cancer detection: fixed-focus and antenna-array sensors. *IEEE Trans. Biomed. Eng.,* 1998, vol. 45, 1470-1479
- **E. C. FEAR ; X. LI ; S. C. HAGNESS ; M. A. STUCHLY.** Confocal microwave imaging for breast cancer detection: Localization of tumors in three dimensions. *IEEE Trans. Biomed. Eng.,* 2002, vol. 49 (8), 812-822
- **R.A.KRUGER ; K.K.KOPECKY ; A.M.AISEN ; D.R.REINECKE ; G.A.KRUGER ; W.L.KISER JR.** Thermoacoustic CT with radio-waves: a medical imaging paradigm. *Radiology,* April 1999, vol. 211 (1), 275-278
- **P. WUST ; B. HILDEBRANDT ; G. SREENIVASA ; B. RAU ; J. GELLERMANN ; H. RIESS ; R. FELIX ; P. M. SCHLAG.** Hyperthermia in combined treatment of cancer. *Lancet Oncol.,* 2002, vol. 3, 487
- **C.BELLOROFONTE ; C.VEDRUCCIO ; P.TOMBOLINI ; M.RUOPPOLO ; A.TUBARO.** Non-invasive detection of prostate cancer by electromagnetic interaction. *European Urology,* 2005, vol. 47, 29-37
- **A.VANNELLI ; E.LEO ; L.BATTAGLIA ; E.POIASINA.** Diagnosis of rectal cancer by electromagnetic interactions: preliminary results. *Desease of the Colon & Rectum,* 2009, vol. 52 (1), 162-166
- **Q.FANG ; P.M.MEANEY ; K.D.PAULSEN.** The multidimensional phase unwrapping integral and applications to microwave tomographical image reconstruction. *IEEE Trans. On Image Processing,* November 2006, vol. 15 (11), 3311-3324
- **H. HINRIKUS ; J.RIIPULK.** Microwave imaging. Wiley Enciclopedia of Biomedical Engineering. J.Wiley and Sons Ed, April 2006